# EUROPEAN PATENT APPLICATION

(11) **EP 2 112 152 A1**
(43) Date of publication of application: **28.10.2009**
(21) Application number: 08103664.2
(22) Date of filing: 22.04.2008
(51) Int. Cl.: C07D 487/04, A61K 31/519, A61P 35/00

(54) **Dihydropteridinones as Plk Inhibitors**

(71) Applicant: GPC Biotech AG, 82152 Martinsried (DE)
(72) Inventor: Brandstetter, Tilmann, 82061, Neuried (DE); Eickhoff, Jan, 80339, München (DE); Köstler, Rolan, 82152, Martinsried (DE); Schoop, Andreas, 82061, Neuried (DE); Sennhenn, Peter, 80539, München (DE); Becker, Frank., 82152 Planegg (DE); Christine, Kauffmann., 81377 München (DE)
(74) Representative: Wallinger, Michael

(57) **Abstract**

The present invention provides derivatives of dihydropteridinone of formula (I). These compounds are inhibitors of Polo-like kinases (Plks), including compounds that show anti-proliferative activity against cells, including against tumor cells, and are useful in the treatment of diseases including cancer.

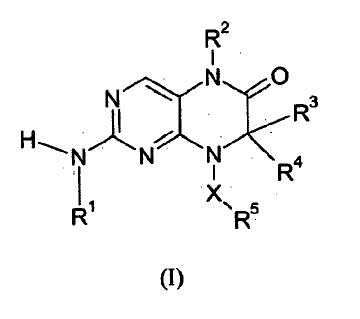

## Description

### Field of the Invention

The present invention provides derivatives of dihydropteridinone. These compounds are inhibitors of kinases such as Polo-like kinases (Plks), including compounds that show anti-proliferative activity against cells, including against tumor cells, and are useful in the treatment of diseases including cancer.

### Background of the Invention

Kinases are important cellular enzymes that perform essential cellular functions such as regulating cell division and proliferation, and appear to play a decisive role in many disease states such as in disease states that are characterized by uncontrolled proliferation and differentiation of cells. These disease states encompass a variety of cell types and maladies such as cancer, atherosclerosis, and restenosis.

Drugs that target kinases, and especially drugs based on small-molecule chemical compounds that inhibit the activity of particular kinases, are important medicines that can significantly improve the health, lifespan or quality of life for people throughout the world, including patients suffering from highly debilitating diseases such as cancer. Indeed, the drug "imatinib" (GLEEVEC/GLIVEC; Novartis) is such a drug that selectively inhibits one kind of kinase and is used to successfully treat certain types of cancer including particular types of leukaemia (CML) and gastrointestinal cancer (GIST). Its improved efficacy compared to previous standard treatments for CML, and that it is generally very well tolerated, showing mild side-effects, has lead, among other reasons, for it to have made the cover of TIME magazine where it was described as a "bullet" to combat cancer.

Polo-like kinases (Plks) are important regulators of cell cycle progression during the M-phase. Named after the polo gene of Drosophila melanogaster, Plks are involved in the assembly and dynamics of the mitotic spindle apparatus and in the activation and inactivation of CDK/cyclin complexes.

Plk1 is a 601 amino acid protein that comprises an N-terminal kinase domain and a C-terminal regulatory domain that contains a sequence comprising 30 amino acid residues that is termed the Polo box. The motif is only observed in the Polo-like kinases. Its function remains unknown. It may be involved in an auto-regulatory mechanism or in targeting the kinase to substrates. Deletion of the C-terminal regulatory domain results in activation of the kinase. Plks have a threonine in the activation segment whose phosphorylation is essential for activity.

Plk1 is principally expressed in G2 and M phases. It localizes at centrosomes in early mitosis, redistributes to the spindle and mitotic bridges at anaphase and is rapidly degraded by the APC/C complex during exit from mitosis. The numerous functions of Plk1 at mitosis are well accounted for by the variety of substrates identified for this kinase, including nucleophosmin (Zhang et al.. (2004) J. Biol. Chem. 279, 35726-35734), Cdc25C phosphatase, cyclin B, a cohesin subunit of the mitotic spindle, subunits of the anaphase promoting complex, and mammalian kinesin-like protein 1 (MKLP-1) and other kinesin related motor proteins (Nigg et al. (1996) Exp Cell Res. 229,174-80). In addition to the regulation of mechanistic aspects of mitosis, Plk1 contributes to set the timing of entry and transition through mitosis by phosphorylating APC/C subunits. Plk1 has been associated with the regulation of the mitotic spindle checkpoint. In addition to its well described roles in mitosis, Plk1 expression in NIH3T3 cells causes the formation of oncogenic foci. Such transformed cells are able to form tumors in nude mice.

Deregulation of Plk1 activity leads to the formation of aberrant centrosomes and mitotic spindle poles. Its overexpression is tightly correlated with the development of chromosomal instability and aneuploidy observed in neoplasias.

Upon selective inhibition of Plk1, mitotic progression is inhibited. Down-regulation of Plk1 by antisense oligonucleotides and siRNA is highly effective in inhibiting cancer-cell proliferation both in vitro and in vivo (Spankuch-Schmitt B. et al (2002) J. Natl. Cancer Inst. 94, 1863-1877; Elez R. et al (2003) Oncogene 22, 69-80). Mitotically arrested cells displayed randomly separated condensed chromosomes and the occurrence of multiple spindle poles with well-formed asters (Schmidt et al (2006) Mol. Cancer Ther. 5, 809-817).

Two additional polo-like kinases with a Polo box have been identified in mammals, Plk2 (Snk) and Plk3 (Prk and Fnk). However, their roles are much less characterized. Plk2 has been shown to act during the G1 / S transition and to be involved in centriole duplication (Warnke et al. (2004) Curr. Biol. 14, 1200-1207). Plk3 has been shown to be required for entry into S phase (Zimmerman and Erikson (2007) Proc. Natl. Acad. Sci. USA 104, 1847-1852). Furthermore, it has been postulated that Plk3 exerts a tumor-suppressive activity (Xie et al. (2001) J. Biol. Chem. 276, 43305-12; Bahassi et al. (2002) Oncogene 21, 6633-40). Additionally, it was shown that Plk1 and Plk3 act synergistically in cell cycle control (Ouyang et al. (1997) J. Biol. Chem. 272; 28646-51). Thus, since the functions of the various members of the Polo-like kinase family are only beginning to be understood in detail, kinase inhibitors that are specific for one or more of the Plks might be advantageous to mitigate off-target effects and hence potentially to diminish drug side-effects, and in any case would prove to be valuable tools useful to further elucidate the biology of the Polo-like kinases.

Despite the fact that the striking biological validation and the potential of Plk1 inhibitors in anticancer therapy has been assumed and described for almost ten years, there has been little progress in the discovery and development of small molecule Plk1 inhibitors. Apart from the compound known as "BI 2536" (see below), there are no Plk1 inhibitors in clinical trials to date. Just recently, the number of preclinical reports on small molecule Plk1 inhibitors increased (McInnes C. et al. (2005) Curr. Topics Med. Chem. 5, 181-197; McInnes C. et al (2006) Nat. Chem. Biol. 608-17 (Epub 2006 Oct 8); Peters U. et al (2006) Nat. Chem. Biol. 618-26 (Epub 2006 Oct 8)).

In vitro and in vivo characterizations of BI 2536 demonstrated specific tumor killing activity of this clinical compound that inhibits Plk1 with high potency (IC₅₀ = 0.83 nM). The compound showed more than 1,000-fold selectivity against a large panel of protein kinases, and also affected the activities of other Polo-like kinase family members, such as Plk2 (IC₅₀ = 3.5 nM) and Plk3 (IC₅₀ = 9 nM). Furthermore, BI 2536 was found to preferentially inhibit cycling cells over resting cells (Steegmaier et al. (2007) Curr. Biol. 17, 316-322; Lénárt et al (2007) Curr. Biol. 17, 304-315).

Thus, despite the progress that has been made, the search continues for low molecular weight kinase inhibitor compounds, especially compounds that inhibit Polo-like kinases such as Plk1, that are useful for treating a wide variety of diseases, including cancer, tumors and other proliferative disorders or diseases including restenosis, angiogenesis, diabetic retinopathy, psoriasis, surgical adhesions, macular degeneration, and atherosclerosis, or other disorders or diseases mentioned herein. Compounds that inhibit a particular kinase or family of kinases, such as Polo-like kinases, without strongly inhibiting other kinases, are particularly sought after as they are presumed to show reduced off-target effects and hence predicted to cause milder side-effects when used as a drug. Thus, a strong need exists to provide compositions, pharmaceuticals and/or medicaments with kinase inhibitory activity, particularly with inhibitory activity against one or more members of the Polo-like kinases such as Plk1, or with anti-proliferative activity against cells such as tumour cells. Particularly, such compositions, pharmaceuticals and/or medicaments that have selective inhibitory activity against certain kinases or a kinase-family such as the Plks, may possess not only such activity, may also exert tolerable, acceptable, milder or diminished side-effects in comparison to other anti-proliferative or anti-cancer agents. Furthermore, the spectrum of tumors or other diseases responsive to treatment with such compositions, pharmaceuticals and/or medicaments may be broad. The active ingredients of such compositions, pharmaceuticals and/or medicaments may be suitable in the mentioned indication as single agent, and/or in combination therapy, be it in connection with other therapeutic agents, with radiation, with operative/surgical procedures, heat treatment or any other treatment known in the mentioned indications.

The potential significance of the Polo-like kinases in the cell-proliferative process, and hence the expected utility of the Plks as drug targets for anti-proliferative agents, thus generates a need to find compounds that are useful to help further elucidate the role and function of the various members of the Plk family. New small-molecule compounds that selective inhibit the Plks compared to other kinases, or in particular show selective inhibition of Plk1 activity, will be valuable tools to use within molecular- and cell-biological research when applied to the understanding of the Polo-like kinases and hence can provide important assistance in the identification of other compounds that could be developed and used as drugs that target Plks, including Plk1.

It is known that specific classes of dihydropteridines, substituted in a specific manner, have pharmacologically useful properties. In particular, specific derivatives of dihydropteridin-6-one, such as BI 2536, are known to possess anti-proliferative activity. These compounds however are structurally dissimilar from the compounds of the present invention.

WO 03/020722 discloses substituted 2-(hetero)arylamino dihydropteridin-6-ones that are shown to be cytotoxic on HeLaS3 cells. Similarly, WO 2004/076454 discloses substituted 2-(4-carboxamidophenylamino)-5-methyl-dihydropteridin-6-ones, including BI 2536.

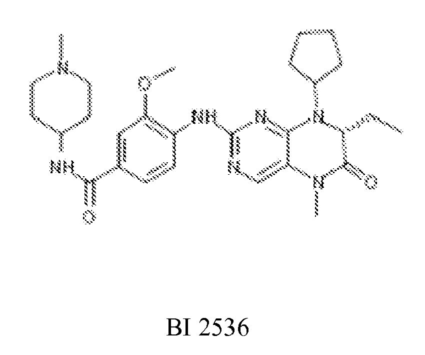

WO 2005/123736 discloses substituted 2-(hetero)arylmethylamino dihydropteridin-6-ones. WO 2006/021378 and WO 2006/021379 disclose substituted 2-(4-acylaminophenylamino)-5-methyl-dihydropteridin-6-ones. WO 2006/021548 discloses substituted 2-(4-aminophenylamino)-5-methyl-dihydropteridin-6-ones and 2-(4-aminopyridylamino)-5-methyl-dihydropteridin-6-ones. WO 2008/009909 discloses substituted 2-(hetero)arylamino-dihydropteridin-6-ones carrying an amino substituent at the nitrogen in position 5. In all these applications, the substituent at the nitrogen at position 8 is either hydrogen or a C-linked substituent, as with BI 2536.

### Summary of the Invention

We have invented a class of 8-substituted dihydropteridin-6-ones that include compounds that exhibit surprising properties, including potent and selective activity as inhibitors of Polo-like kinases, in particular potent inhibition of Plk1, or anti-proliferative activity against cells such as tumour cells. Such derivatives of dihydropteridin-6-one provide an opportunity to develop new and effective therapies for diseases associated with kinase de-regulation, especially de-regulation of Polo-like kinases, or cellular proliferation, such as cancer or inflammatory disorders. The selective inhibition of the activity of one or more Polo-like kinases compared to kinase from other families, may lead to the development and use of compounds of the invention as drugs with less off-target effects, and potentially showing milder or diminished treatment side-effects compared to alternative therapies. Furthermore, the compounds of the invention being selective inhibitors of one or more Polo-like kinases, can be used as valuable tools within molecular- and cellular-biological research to better understand the role and function of Plks, which in turn will be of importance in the identification of additional compounds that inhibit this important class of drug-targets.

Compounds of the present invention are specific derivatives of dihydropteridin-6-one, as discussed in greater detail below. In analogy to certain dihydropteridin-6-ones previously described, the compounds of the present invention are suitable for further pre-clinical or clinical research and development towards their use as drugs for the treatment of a variety of disorders and diseases including cancer, proliferative, degenerative, inflammatory and other disorders and diseases. The invention, useful as either drug-candidates or as tools in research into Polo-like kinases, can lead to effective therapies and therapeutics for particularly debilitating diseases such as cancer and other diseases and disorders including those listed herein.

One aspect of the invention relates to 8-substituted dihydropteridin-6-ones having a structure represented by formula (I) presented below, or tautomeric or stereoisomeric forms thereof, which are useful as kinase inhibitors, especially as inhibitors of one or more Polo-like kinases such as Plk1, and thus useful for treating proliferative disorders or diseases such as such as cancer, atherosclerosis, and restenosis, among others, or are useful as selective Plk inhibitors to further elucidate the function of Polo-like kinases

In another aspect, the invention relates to pharmaceutical compositions, including a pharmaceutically acceptable diluent, excipient or carrier and an amount, such as a therapeutically effective amount, of such kinase inhibitor, e.g., which is expected to ameliorate the effects of proliferative disorders or diseases including those mentioned herein, such as cancer.

Another aspect of the invention relates to a pharmaceutical package, including such pharmaceutical composition, and instructions which indicate that said pharmaceutical composition may be used for the treatment of a patient suffering from a proliferative disorder or disease, including those mentioned herein, such as cancer.

In another aspect, the invention relates to methods that involve administering to or contacting a subject, a cell, a tissue, an organ or an organism with a therapeutically effective amount of a compound or pharmaceutical composition disclosed herein. These methods include, but are not limited to, prophylaxis and/or treatment of a proliferative disorder or disease including those mentioned herein, such as cancer.

In another aspect, the invention relates to uses of the compounds of the present invention for the preparation of a medicament for the treatment of a proliferative disorder or disease, including those mentioned herein, such as cancer.

Another aspect of the invention related to the use of the compounds of the present invention for the preparation of a composition for the inhibition of one or more Polo-like kinase, and the use of such compound or composition to inhibit such kinase, including the inhibition of Plk1.

Another aspect of the invention relates to methods of synthesizing the compounds of the present invention, and to intermediates for such compounds.

Accordingly, the present invention provides compounds having a structure represented by the general formula (I) 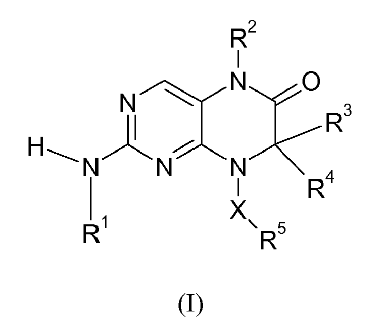 or any tautomeric or stereoisomeric form thereof, wherein
R¹ is selected from substituted or unsubstituted: -aryl and -heteroaryl;
R² is selected from hydrogen, -CH(=O), -OH, substituted or unsubstituted -O-C₁₋₃-alkyl and substituted or unsubstituted -C₁₋₆-alkyl;
R³ and R⁴ are independently selected from hydrogen, and substituted or unsubstituted: - C₁₋₁₀-alkyl, -C₂₋₁₀-alkenyl, -C₂₋₁₀-alkynyl, -C₃₋₁₀-cycloalkyl, -C₄₋₁₀-cycloalkenyl, heterocycloalkyl, heterocycloalkenyl, aryl, and heteroaryl;
   alternatively, R³ and R⁴, together with the carbon atom to which they are attached, form a substituted or unsubstituted: C₃₋₈-cycloalkyl, C₄₋₈-cycloalkenyl or a substituted or unsubstituted: 3- to 8-membered heterocycloalkyl or heterocycloalkenyl comprising 1, 2 or 3 heteroatoms selected from N and O;
X is selected from -O- and -NR⁶;
R⁵ and R⁶ are independently selected from hydrogen, and substituted or unsubstituted: - C₁₋₆-alkyl, -C₂₋₆-alkenyl, -C₂₋₆-alkynyl, -C₃₋₆-cycloalkyl and -C₄₋₆-cycloalkenyl;
   alternatively, for X = NR⁶, R⁵ and R⁶, together with the nitrogen atom they are attached to, form a substituted or unsubstituted 3- to 8-membered heterocycloalkyl or heterocycloalkenyl comprising 0, 1 or 2 additional heteroatoms selected from N and O;
   alternatively, one of R³ and R⁴, and R⁵, together with the group -O-N-C-, or -N-N-C, respectively, to which they are attached, form a substituted or unsubstituted: 3- to 8-membered heterocycloalkyl or heterocycloalkenyl comprising 0 or 1 additional heteroatom selected from N and O;
   or any pharmaceutically acceptable salt or N-oxide thereof.

Other features and advantages of the invention will be apparent from the following detailed description and from the claims.

### Detailed Description of the Invention

### Definitions

The term "alkyl" refers to straight- or branched-chain saturated hydrocarbon groups having from 1 to about 20 carbon atoms, including groups having from 1 to about 7 carbon atoms. In certain embodiments, alkyl substituents may be lower alkyl substituents. The term "lower alkyl" refers to alkyl groups having from 1 to 6 carbon atoms, and in certain embodiments from 1 to 4 carbon atoms. Examples of alkyl groups include, but are not limited to, methyl, ethyl, n-propyl, i-propyl, n-butyl, s-butyl, t-butyl, n-pentyl, s-pentyl, and n-hexyl.

The term "alkenyl" refers to groups having 2 to about 20 carbon atoms, wherein at least one of the carbon-carbon bonds is a double bond, while other bonds may be single bonds or further double bonds. The term "alkynyl" herein refers to groups having 2 to about 20 carbon atoms, wherein at least one of the carbon-carbon bonds is a triple bond, while other bonds may be single, double or further triple bonds. Examples of alkenyl groups include ethenyl, 1-propenyl, 2-propenyl, 1-butenyl, 2-butenyl, 3-butenyl, 2-methyl-1-propenyl, 2-methyl-2-propenyl, and the like. Examples of alkynyl groups include ethynyl, 1-propynyl, 2-propynyl, and so forth.

As used herein, "cycloalkyl" is intended to refer to a ring being part of any stable monocyclic or polycyclic system, where such ring has between 3 and about 12 carbon atoms, but no heteroatom, and where such ring is fully saturated, and the term "cycloalkenyl" is intended to refer to a ring being part of any stable monocyclic or polycyclic system, where such ring has between 3 and about 12 carbon atoms, but no heteroatom, and where such ring is at least partially unsaturated (but excluding any aryl ring). Examples of cycloalkyls include, but are not limited to, cyclopropyl, cyclobutyl, cyclopentyl, cyclohexyl, cycloheptyl, adamantyl, cyclooctyl, bicycloalkyls, including bicyclooctanes such as [2.2.2]bicyclooctane, bicyclononanes such as [4.3.0]bicyclononane, and bicyclodecanes such as [4.4.0]bicyclodecane (decalin), or spiro compounds. Examples of cycloalkenyls include, but are not limited to, cyclopentenyl or cyclohexenyl. For the sake of clarity, if a substituent is a polycyclic ring system as described above wherein one ring is a least partially unsaturated, then such substituent will be referred to as "cycloalkenyl", if substitution occurs via the at least partially unsaturated ring, and as "cycloalkyl", if substitution occurs via a fully saturated ring.

The term "C_{x-y}-", when used in combination with a group as defined herein, is intended to indicate the range of carbon atoms being present in the respective group, excluding substituents. For example, the term "C₁₋₆-alkyl" refers to the alkyl groups having between one carbon atom (i.e. a methyl group) and six carbon atoms (e.g. n-hexyl); the term "C₃₋₆-cycloalkyl" " refers to cycloalkyl groups having between three carbon atom (i.e. a cyclopropyl group) and six carbon atoms (i.e.. cyclohexyl).

As used herein, the terms "heterocycloalkyl" and "heterocycloalkenyl", are intended to refer to a ring being part of any stable monocyclic or polycyclic ring system, where such ring has between 4 and about 12 atoms, and where such ring consists of carbon atoms and at least one heteroatom, particularly at least one heteroatom independently selected from the group consisting of N, O and S, with heterocycloalkyl referring to such a ring that is fully saturated, and heterocycloalkenyl referring to a ring that is at least partially unsaturated (but excluding any aryl or heteroaryl ring). For the sake of clarity, if a substituent is a polycyclic ring system wherein one ring contains at least one heteroatom as described herein, then such substituent will be referred to as "heterocycloalkyl/-alkenyl", if substitution occurs via the ring containing the heteroatom(s). Heterocycloalkyl and heterocycloalkenyl groups may be linked to other groups via a carbon ring atom ("C-linked heterocycloalkyl" and "C-linked heterocycloalkenyl", respectively), or via a nitrogen ring atom ("N-linked heterocycloalkyl" and "N-linked heterocycloalkenyl", respectively). Heteroatoms such as nitrogen and sulfur may optionally be oxidized to form N-oxides or sulfoxides and sulfones, respectively. In certain embodiments, a nitrogen in the heterocycle may be quaternized. In certain embodiments, where the total number of S and O atoms in the heterocycle exceeds 1, these heteroatoms are not adjacent to one another. In particular embodiments, the total number of S atoms in the heterocycle is not more than 1.

Examples of heterocycloalkyls include, but are not limited to, pyrrolidinyl, tetrahydrofuranyl, morpholinyl, thiomorpholinyl, piperazinyl, piperidinyl, and decahydroquinolinyl (when linked via the piperidinyl moiety). Examples of heterocycloalkenyls include, but are not limited to, pyrrolinyl, and indolenyl (when linked via the 5-membered ring). Also included are fused ring systems sand spiro compounds containing, for example, any of the above heterocycles, in each case when linked a heteratom-containing ring.

The term "aryl" is intended to mean a ring or ring system being part of any stable monocyclic or polycyclic system, where such ring or ring system has between 3 and about 20 carbon atoms, but has no heteroatom, and where the ring or ring system consists of an aromatic moiety as defined by the "(4n+2) π electron rule". For the sake of clarity, if a substituent is a polycyclic system wherein one ring or ring system comprised in said polycyclic system consists of an aromatic moiety as defined herein, then such substituent will be referred to as "aryl", if substitution occurs via said aromatic moiety. This includes, but is not limited to, phenyl and fused benzene ring systems, for example, naphthalene, anthracene, or phenanthrene ring systems, or, for example, a benzene ring fused to one or more cycloalkyl moieties to form, for example, indanyl, fluorenyl or tetrahydronaphthyl (tetralin), or fused to one or more heterocycloalklyl rings, e.g. as in indolenyl, *provided*, *however*, that in each such case, such fused system is linked as a substituent via the aromatic moiety.

As used herein, the term "heteroaryl" refers to a ring or ring system being part of any stable mono- or polycyclic system, where such ring or ring system has between 3 and about 20 atoms, which ring or ring system consists of an aromatic moiety as defined by the "(4n+2) π electron rule" and which contains carbon atoms and one or more nitrogen, sulfur, and/or oxygen heteroatoms. For the sake of clarity, if a substituent is a polycyclic system wherein one ring or ring system comprised in said polycyclic system consists of an aromatic moiety containing a heteroatom as defined herein, then such substituent will be referred to as "heteroaryl", if substitution occurs via the aromatic moiety containing the heteroatom. In certain embodiments, the total number of N, S and O atoms in the heteroaryl is between 1 and about 4. In certain embodiments, the total number of S and O atoms in the aromatic heteroaryl is not more than 1. In certain embodiments, a nitrogen in the heterocycle may be quaternized or oxidized to an N-oxide. Examples of heteroaryls include, but are not limited to, pyrrolyl, pyrazolyl, imidazolyl indolyl, benzimidazolyl, furanyl, benzofuranyl, thiophenyl, benzothiophenyl, pyridinyl, pyrimidinyl, pyrazinyl, triazinyl, quinolinyl, quinazolinyl.

Also included in the term heteroaryl are fused heteroaryls containing, for example, the above heteroaryls fused to cycloalkyls or heterocycloalkyls (provided, in each case, that such fused system is linked as a substituent via the aromatic moiety containing at least one heteroatom).

Wherever indicated herein, alkyl, alkenyl, alkynyl, cycloalkyl, cycloalkenyl, heterocycloalkyl, heterocycloalkenyl, aryl and heteroaryl groups as well as and any other substructure comprising at least one hydrogen in the substructure may be substituted by one or more substituents.

"Substituted" is intended to indicate that one or more hydrogens on the atom or group indicated in the expression using "substituted" is replaced with a selection from the indicated group(s), provided that the indicated atom's normal valency, or that of the appropriate atom of the group that is substituted, is not exceeded, and that the substitution results in a stable compound. The terms "substituted or unsubstituted" or "optionally substituted" are intended to mean that a given compound, or substructure of a compound, is either unsubstituted, or substituted, as defined herein, with one or more substituents, as indicated or as defined below.

In a substituted moiety, unless a specific list of substituents is explicitly provided, at least one hydrogen atom of the moiety "M" to be substituted is replaced by a substituent -[(R^{Sm})ₙ], wherein m is an integer running from 1 to n, wherein n is any value from 1 to about 15, wherein each R^{Sm} is independently selected from the following lists (A) and (B):
(A): alkyl, alkenyl, alkynyl, cycloalkyl, cycloalkenyl, C-linked heterocycloalkyl, C-linked heterocycloalkenyl, aryl, C-linked heteroaryl, -O-R^{a}, -O-N(R^{a})₂, -S-R^{a}, -S-N(R^{a})₂, N-linked heterocycloalkyl, N-linked heteroaryl, -N(R^{a})-R^{a}, -N(R^{a})-OR^{a}, -N(R^{a})-SR^{a}, -N(R^{a})-N(R^{a})₂, -F, -Cl, -Br, -I;
(B): -C(=O)-R^{a}, -C(=S)-R^{a}, -C(=NR^{a})-R^{a}, -C(=N-OR^{a})-R^{a}, -C(=N-N(R^{a})₂)-R^{a}, -C(=O)-OR^{a}, -C(=S)-OR^{a}, -C(=O)-N(R^{a})₂, -C(=S)-N(R^{a})₂, -C(=NR^{a})-N(R^{a})₂, -C(=N-OR^{a})-N(R^{a})₂, - C(=N-N(R^{a})₂)-N(R^{a})₂, -C(=O)-N(R^{a})-N(R^{a})₂, -C(=S)-N(R^{a})-N(R^{a})₂, -C(=NR^{a})-N(R^{a})-N(R^{a})₂, - CN, -O-N=C(R^{a})₂, -O-C(=O)-R^{a}, -O-C(=S)-R^{a}, -O-C(=NR^{a})-R^{a}, -O-C(=N-OR^{a})-R^{a}, -O-C(=N-N(R^{a})₂)-R^{a}, -O-C(=NR^{a})-OR^{a}, -O-C(=NR^{a})-SR^{a}, -O-C(=NR^{a})-N(R^{a})₂, -S-N(R^{a})-N(R^{a})₂, -S-N=C(R^{a})₂, -S-N=NR^{a}, -SO-R^{a}, -SO-OR^{a}, -SO-SR^{a}, -SO-N(R^{a})₂, -SO-N(R^{a})-N(R^{a})₂, -SO₂-R^{a}, - SO₂-OR^{a}, -SO₂-SR^{a}, -SO₂-N(R^{a})₂, -SO₂-N(R^{a})-N(R^{a})₂, -N(R^{a})-N=C(R^{a})₂, -N(R^{a})-C(=O)-R^{a}, - N(R^{a})-C(=S)-R^{a}, -N(R^{a})-C(=NR^{a})-R^{a}, -N(R^{a})-C(=N-OR^{a})-R^{a}, -N(R^{a})-C(=N-N(R^{a})₂)-R^{a}, -N(R^{a})-C(=O)-OR^{a}, -N(R^{a})-C(=S)-OR^{a}, -N(R^{a})-C(=NR^{a})-OR^{a}, -N(R^{a})-C(=N-OR^{a})-OR^{a}, -N(R^{a})-C(=N-N(R^{a})₂)-OR^{a}, -N(R^{a})-C(=O)-N(R^{a})₂, -N(R^{a})-C(=S)-N(R^{a})₂, -N(R^{a})-C(=NR^{a})-N(R^{a})₂, -N(R^{a})-C(=N-OR^{a})-N(R^{a})₂, -N(R^{a})-C(=N-N(R^{a})₂)-N(R^{a})₂, -N(R^{a})-C(=O)-N(R^{a})-N(R^{a})₂, -N(R^{a})-C(=S)-N(R^{a})-N(R^{a})₂, -N(R^{a})-C(=NR^{a})-N(R^{a})-N(R^{a})₂, -N(R^{a})-C(=N-OR^{a})-N(R^{a})-N(R^{a})₂, -N(R^{a})-C(=N-N(R^{a})₂)-N(R^{a})-N(R^{a})₂, -N(R^{a})-N(R^{a})-C(=O)-R^{a}, -N(R^{a})-N(R^{a})-C(=S)-R^{a}, -N(R^{a})-N(R^{a})-C(=NR^{a})-R^{a}, -N(R^{a})-N(R^{a})-C(=N-OR^{a})-R^{a}, -N(R^{a})-N(R^{a})-C(=N-N(R^{a})₂)-R^{a}, -N(R^{a})-N(R^{a})-C(=O)-OR^{a}, -N(R^{a})-N(R^{a})-C(=S)-OR^{a}, -N(R^{a})-N(R^{a})-C(=NR^{a})-OR^{a}, -N(R^{a})-N(R^{a})-C(=N-OR^{a})-OR^{a}, -N(R^{a})-N(R^{a})-C(=N-N(R^{a})₂)-OR^{a}, -N(R^{a})-N(R^{a})-C(=O)-N(R^{a})₂, -N(R^{a})-N(R^{a})-C(=S)-N(R^{a})₂, -N(R^{a})-N(R^{a})-C(=NR^{a})-N(R^{a})₂, -N(R^{a})-N(R^{a})-C(=N-OR^{a})-N(R^{a})₂, -N(R^{a})-N(R^{a})-C(=N-N(R^{a})₂)-N(R^{a})₂, -N(R^{a})-N(R^{a})-C(=O)-N(R^{a})-N(R^{a})₂, -N(R^{a})-N(R^{a})-C(=S)-N(R^{a})-N(R^{a})₂, -N(R^{a})-N(R^{a})-C(=NR^{a})-N(R^{a})-N(R^{a})₂, -N(R^{a})-N(R^{a})-C(=N-OR^{a})-N(R^{a})-N(R^{a})₂, -N(R^{a})-N(R^{a})-C(=N-N(R^{a})₂)-N(R^{a})-N(R^{a})₂, -N(R^{a})-N(R^{a})-C(=NR^{a})-N=C(R^{a})_{2,} -N(R^{a})-N(R^{a})-C(=N-OR^{a})-N=C(R^{a})₂, -N(R^{a})-N(R^{a})-C(=N-N(R^{a})₂)-N=C(R^{a})₂, -N(R^{a})-SO-R^{a}, -N(R^{a})-SO-OR^{a}, -N(R^{a})-SO-N(R^{a})₂, - N(R^{a})-SO-NR^{a}-N(R^{a})₂, -N(R^{a})-SO₂-R^{a}, -N(R^{a})-SO₂-OR^{a}, -N(R^{a})-SO₂-N(R^{a})₂, -N(R^{a})-SO₂-NR^{a}-N(R^{a})₂, -NO₂, -N₃, -F, -Cl, -Br, -I;
wherein each R^{a} is independently selected from -H and R^{b}, with R^{b} being independently selected from alkyl, alkenyl, alkynyl, cycloalkyl, cycloalkenyl, C-linked heterocycloalkyl, C-linked heterocycloalkenyl, aryl, and C-linked heteroaryl, and wherein alternatively two substituents R^{b}, when attached to the same nitrogen atom, can form together with the nitrogen atom they are attached to a heterocycloalkyl, heterocycloalkenyl, or heteroaryl group,
and wherein R^{S1} is attached to such moiety M, and wherein any additional R^{Sx}, if present, is substituting a hydrogen atom in a residue R^{b} of a group R^{Sy}, with x > y.

A methylene group present in a ring of a cycloalkyl, cycloalkenyl, heterocycloalkyl, and heterocycloalkenyl moiety may as well be substituted by a substituent R^{S} taken from the following list (C):
(C): =O, =S, =NR^{a}, =N-OR^{a}, and =N-N(R^{a})₂, wherein for each such substituent both hydrogens on the methylene group of the unsubstituted moiety are being replaced.

Nitrogen-containing substructures, such as amines or nitrogen-containing heterocycles, may be substituted as well by formation of quaternary amines or N-oxides, wherein R^{S} is alkyl or -O.

For example, a substituent -CH₂-O-CH₂-CH₂-F can be described as -[(R^{Sm})₃] with R^{S3} being -F that substitutes a hydrogen atom in R^{S2} being -O-C₂-alkyl, where such R^{S2} substitutes a hydrogen atom in R^{S1} being C₁-alkyl. In this example, the individual elements R^{Sm} in -[(R^{Sm})₃] are arranged in a "linear" fashion, since every R^{Sx} is linked to a R^{Sy} with x = y +1 (i.e. R^{S3} is linked to R^{S2}, and so on).

As a second example, the substituent -CH₂-O-CH₂-CF₃ can be described as -[(R^{Sm})₅] with each of R^{S3}, R^{S4}, and R^{S5} being a -F atom that substitutes a hydrogen atom in R^{S2} being -O-C₂-alkyl, where such R^{S2} substitutes a hydrogen atom in R^{S1} being C₁-alkyl. In this example, the individual elements R^{Sm} in -[(R^{Sm})₆] are arranged in a "branched" fashion, since at least one R^{Sx} (here, for example, R^{S4} or R^{S5}) is linked to a R^{Sy} (here R^{S2}) with x > y +1.

As will be readily apparent to anyone of ordinary skill in the art, there are manifold combinations of individual elements R^{S} possible. However, as is apparent to one of ordinary skill in the art as well, there are certain combinations that result in a less favored arrangement and that will not be considered in the design and synthesis of compounds in accordance with the present invention, because of low stability, high reactivity, or unfavorable physicochemical properties, such as low solubility. Examples include, but are not limited to, semi-acetals or -ketals (-O-CR₂-OH), Michael systems (e.g. -C(=O)-CR=CR₂), α-halogen carbonyl compounds (e.g. -C(=O)-CR₂-Br), polycyclic aromatic systems, and the like.

In particular embodiments, the number of elements R^{S} in a substituent -[(R^{Sm})ₙ], that are selected from the lists (B) and (C) listed above, is not more than two. In particular embodiments, there is not more than one element R^{S} in a substituent -[(R^{Sm})ₙ], that is selected from the lists (B) or (C) listed above.

In particular embodiments, n in a substituent -[(R^{Sm})ₙ] is any value from 1 to about 12, from 1 to about 10, from 1 to about 8, or from 1 to about 6.

In particular embodiments, n in a substituent -[(R^{Sm})ₙ] for all substituents different from -F or -Cl is any value from 1 to about 6, from 1 to about 5, from 1 to about 4, or from 1 to about 3.

In particular embodiments, the number of atoms in a substituent -[(R^{Sm})ₙ] is any value from 1 to about 80, from 1 to about 60, from 1 to about 50, from 1 to about 40, from 1 to about 30, from 1 to about 20, from 1 to about 15, or from 1 to about 10.

In particular embodiments, the combined molecular weight of all atoms in a substituent - [(R^{Sm})ₙ] is any value from 1 to about 360, from 1 to about 300, from 1 to about 240, from 1 to about 180, from 1 to about 120, from 1 to about 90, or from 1 to about 60.

The term "halogen" or "halo" refers to fluoro, chloro, bromo and iodo substituents.

The terms "stereoisomer" and "tautomer" as used herein include all possible stereoisomeric and tautomeric forms of the compounds of the present invention. Where the compounds of the present invention contain one or more chiral centers, all possible enantiomeric and diastereomeric forms are included.

The present invention is intended to include all isotopes of atoms occurring on the present compounds. Isotopes are atoms having the same atomic number but different mass numbers. By way of general example and without limitation, isotopes of hydrogen include tritium and deuterium. Isotopes of carbon include ¹²C and ¹⁴C.

The term "metabolite", as used herein, refers to any substance produced by the metabolism or by a metabolic process. Metabolism, as used herein, refers to the various physical/chemical/biochemical/pharmacological reactions involved in the transformation of molecules or chemical compounds occurring in the cell, tissue, system, body, animal, individual, patient or human therein.

The term "IC₅₀", as used herein, refers to concentrations at which a measurable activity, phenotype or response, for example growth or proliferation of cells such as tumor cells, is inhibited by 50%. IC₅₀ values can be estimated from an appropriate dose-response curve, for example by eye or by using appropriate curve fitting or statistical software. More accurately, IC₅₀ values may be determined using non-linear regression analysis.

As used herein, an "individual" means a multi-cellular organism, for example an animal such as a mammal, including a primate. In addition to primates, such as humans, a variety of other mammals can be treated according to a method that utilizes one or more compounds of the present invention. For example, mammals including, but not limited to, cows, sheep, goats, horses, dogs, cats, guinea pigs, rats or other bovine, ovine, equine, canine, feline, rodent, or murine species can be used.

As used herein, a "proliferative disorder" or a "proliferative disease" includes a disease or disorder that affects a cellular growth, differentiation, or proliferation process.

As used herein, a "cellular growth, differentiation or proliferation process" is a process by which a cell increases in number, size or content, by which a cell develops a specialized set of characteristics which differ from that of other cells, or by which a cell moves closer to or further from a particular location or stimulus. A cellular growth, differentiation, or proliferation process includes amino acid transport and degradation and other metabolic processes of a cell. A cellular proliferation disorder may be characterized by aberrantly regulated cellular growth, proliferation, differentiation, or migration. Cellular proliferation disorders include tumorigenic diseases or disorders.

As used herein, a "tumorigenic disease or disorder" includes a disease or disorder characterized by aberrantly regulated cellular growth, proliferation, differentiation, adhesion, or migration, which may result in the production of or tendency to produce tumors. As used herein, a "tumor" includes a benign or malignant mass of tissue. Examples of cellular growth or proliferation disorders include, but are not limited to tumors, cancer, autoimmune diseases, viral diseases, fungal diseases, neurodegenerative disorders and cardiovascular diseases.

As used herein, the terms "anti-cancer agent" or "anti-proliferative agent" refer to compounds with anti-cancer and anti-proliferative properties, respectively. These compounds include, but are not limited to, altretamine, busulfan, chlorambucil, cyclophosphamide, ifosfamide, mechlorethamine, melphalan, thiotepa, cladribine, fluorouracil, floxuridine, gemcitabine, thioguanine, pentostatin, methotrexate, 6-mercaptopurine, cytarabine, carmustine, lomustine, streptozotocin, carboplatin, cisplatin, oxaliplatin, picoplatin, LA-12, iproplatin, tetraplatin, lobaplatin, JM216, JM335, satraplatin, fludarabine, aminoglutethimide, flutamide, goserelin, leuprolide, megestrol acetate, cyproterone acetate, tamoxifen, anastrozole, bicalutamide, dexamethasone, diethylstilbestrol, prednisone, bleomycin, dactinomycin, daunorubicin, doxirubicin, idarubicin, mitoxantrone, losoxantrone, mitomycin-c, plicamycin, paclitaxel, docetaxel, topotecan, irinotecan, 9-amino camptothecan, 9-nitro camptothecan, GS-211, JM 118, etoposide, teniposide, vinblastine, vincristine, vinorelbine, procarbazine, asparaginase, pegaspargase, octreotide, estramustine, and hydroxyurea. Said terms also include, but are not limited to, non-small molecule therapeutics, such as antibodies, e.g., 1D09C3 and other anti-HLA-DR antibodies as described in WO 01/87337 and WO 01/97338, Rituxan as described in US patents 5,736,137, 5,776,456, 5,843,437, 4D5, Mab225, C225, Daclizumab (Zenapax), Antegren, CDP 870, CMB-401, MDX-33, MDX-220, MDX-477, CEA-CIDE, AHM, Vitaxin, 3622W94, Therex, 5G1.1, IDEC-131, HU-901, Mylotarg, Zamyl (SMART M195), MDX-210, Humicade, LymphoCIDE, ABX-EGF, 17-1A, Trastuzumab (Herceptin ®, rhuMAb), Epratuzumab, Cetuximab (Erbitux ®), Pertuzumab (Omnitarg®, 2C4), R3, CDP860, Bevacizumab (Avastin ®), tositumomab (Bexxar ®), Ibritumomab tiuxetan (Zevalin ®), M195, 1D10, HulD10 (Remitogen®, apolizumab), Danton/DN1924, an "HD" antibody such as HD4 or HD8, CAMPATH-1 and CAMPATH-1H or other variants, fragments, conjugates, derivatives and modifications thereof, or other equivalent compositions with improved or optimized properties, and proteins or peptides, e.g., those described in Trends in Biotechnology (2003), 21(12), p.556-562.

As used herein, an "inflammatory disorder" or an "inflammatory disease" includes a disease or disorder that is caused or accompanied by inflammatory processes. This includes, but is not limited to, diseases or disorders such as arthritis, including but not limited to rheumatoid arthritis, spondyloarthropathies, gouty arthritis, osteoarthritis, systemic lupus erythematosus and juvenile arthritis, osteoarthritis, gouty arthritis and other arthritic conditions; pulmonary disorders or lung inflammation, including adult respiratory distress syndrome, pulmonary sarcoidosis, asthma, silicosis, and chronic pulmonary inflammatory disease; viral and bacterial infections, including sepsis, septic shock, gram negative sepsis, malaria, meningitis, cachexia secondary to infection or malignancy, cachexia secondary to acquired immune deficiency syndrome (AIDS), AIDS, ARC (AIDS related complex), pneumonia, and herpes virus; bone resorption diseases, such as osteoporosis, endotoxic shock, toxic shock syndrome, reperfusion injury, autoimmune disease including graft vs. host reaction and allograft rejections, cardiovascular diseases including atherosclerosis, thrombosis, congestive heart failure, and cardiac reperfusion injury, renal reperfusion injury, liver disease and nephritis, and myalgias due to infection; Alzheimer's disease, influenza, multiple sclerosis, cancer, diabetes, systemic lupus erythematosus (SLE), skin-related conditions such as psoriasis, eczema, bums, dermatitis, keloid formation, and scar tissue formation; gastrointestinal conditions such as inflammatory bowel disease, Crohn's disease, gastritis, irritable bowel syndrome and ulcerative colitis; ophthalmic diseases, such as retinitis, retinopathies, uveitis, ocular photophobia, and of acute injury to the eye tissue; angiogenesis, including neoplasia; metastasis; ophthalmological conditions such as corneal graft rejection, ocular neovascularization, retinal neovascularization including neovascularization following injury or infection, diabetic retinopathy, retrolental fibroplasia and neovascular glaucoma; ulcerative diseases such as gastric ulcer; pathological, but non-malignant, conditions such as hemangiomas, including infantile hemangiomas, angiofibroma of the nasopharynx and avascular necrosis of bone; diabetic nephropathy and cardiomyopathy; and disorders of the female reproductive system such as endometriosis.

As used herein, "pharmaceutically acceptable salts" refers to derivatives of the disclosed compounds wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. The pharmaceutically acceptable salts include the conventional non-toxic salts or the quaternary ammonium salts of the parent compound formed, for example, from non-toxic inorganic or organic acids. For example, such conventional non-toxic salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, palmoic, maleic, hydroxymaleic, phenylacetic, glutamic, benzoic, salicylic, sulfanilic, 2-acetoxybenzoic, fumaric, toluenesulfonic, methanesulfonic, ethane disulfonic, oxalic, isethionic, and the like.

The pharmaceutically acceptable salts of the present invention can be synthesized from a parent compound which contains a basic or acidic moiety by conventional chemical methods. Generally, such salts can be prepared by reacting the free acid or base forms of these compounds with a stoichiometric amount of the appropriate base or acid in water or in an organic solvent, or in a mixture of the two; generally, nonaqueous media like ether, EtOAc, ethanol, isopropanol, or acetonitrile are preferred. Lists of suitable salts are found in Remington's Pharmaceutical Sciences, 18th ed., Mack Publishing Company, Easton, PA, 1990, p. 1445, the disclosure of which is hereby incorporated by reference.

Any salt that retains the desired biological activity of the compounds contained herein and that exhibits minimal or no undesired or toxicological effects is intended for inclusion here. Pharmaceutically acceptable salts include those derived from pharmaceutically acceptable organic or inorganic acids and bases. Non-pharmaceutically acceptable acids and bases also find use herein, as for example, in the synthesis and/or purification of the compounds of interest. Thus, all "salts" are also encompassed within the scope of the instant invention.

Non-limiting examples of suitable salts include those derived from inorganic acids, such as, for example, hydrochloric acid, hydrobromic acid, sulfuric acid, phosphoric acid, nitric acid, bicarbonic acid, carbonic acid; and salts formed with organic acids, such as, for example, formic acid, acetic acid, oxalic acid, tartaric acid, succinic acid, malic acid, malonic acid, ascorbic acid, citric acid, benzoic acid, tannic acid, palmoic acid, alginic acid, polyglutamic acid, tosic acid, methanesulfonic acid, naphthalenesulfonic acid, naphthalenedisulfonic acid, α-ketoglutaric acid, β-glycerophosphoric acid and polygalacturonic acid. Suitable salts include those derived from alkali metals such as lithium, potassium and sodium, from alkaline earth metals such as calcium and magnesium, as well as from other acids well known to those of skill in the pharmaceutical art. Other suitable salts include those derived from metal cations such as zinc, bismuth, barium, or aluminum, or with a cation formed from an amine, such as ammonia, N,N-dibenzylethylenediamine, D-glucosamine, tetraethylammonium, or ethylenediamine. Moreover, suitable salts include those derived from a combination of acids and bases, such as, for example, a zinc tannate salt.

The phrase "pharmaceutically acceptable" is employed herein to refer to those compounds, materials, compositions, and/or dosage forms which are, within the scope of sound medical judgment, suitable for use in contact with the tissues of human beings and animals without excessive toxicity, irritation, allergic response, or other problem or complication commensurate with a reasonable benefit/risk ratio.

The term "prodrug", as used herein, refers to an agent that is converted into a pharmacologically active parent drug in vivo, such as a compound as defined herein. The term "prodrug" includes any covalently bonded carriers that release an active parent drug of the present invention in vivo when such prodrug is administered to an animal. Since prodrugs are known to enhance numerous desirable qualities of pharmaceuticals (e.g., solubility, bioavailability, manufacturing, transport, pharmacodynamics, etc.), the compounds of the present invention may be delivered in prodrug form. Prodrugs, for instance, may be bioavailable by oral administration even when the parent drug is not. Thus, the present invention is intended to cover prodrugs of the presently claimed compounds, methods of delivering the same, and compositions containing the same. Prodrugs of the present invention are prepared by modifying functional groups present in the compound in such a way that the modifications are cleaved, either in routine manipulation or in vivo, to the parent compound. Prodrugs include compounds of the present invention wherein a hydroxy, amino, or sulfhydryl group is bonded to any group that, when the prodrug of the present invention is administered to a mammalian subject, it cleaves to form a free hydroxyl, free amino, or free sulfhydryl group, respectively. Examples of prodrugs include, but are not limited to, acetate, formate, and benzoate derivatives of alcohol and amine functional groups in the compounds of the present invention.

Generally speaking, prodrugs are derivatives of *per se* drugs that after administration undergo conversion or metabolism to the physiologically active species. The conversion may be spontaneous, such as hydrolysis in the physiological environment, or may be enzyme-catalyzed. Prodrugs include compounds that can be oxidized, reduced, aminated, deaminated, hydroxylated, dehydroxylated, hydrolyzed, esterified, alkylated, dealkylated, acylated, deacylated, phosphorylated, and/or dephosphorylated to produce the active compound.

From among the voluminous scientific literature devoted to prodrugs in general, the foregoing examples are cited: Gangwar et al., "Prodrug, molecular structure and percutaneous delivery", Des. Biopharm. Prop. Prodrugs Analogs, [Symp.] Meeting Date 1976, 409-21. (1977); Nathwani and Wood, "Penicillins: a current review of their clinical pharmacology and therapeutic use", Drugs 45(6): 866-94 (1993); Sinhababu and Thakker, "Prodrugs of anticancer agents", Adv. Drug Delivery Rev. 19(2): 241-273 (1996); Stella et al., "Prodrugs. Do they have advantages in clinical practice?", Drugs 29(5): 455-73 (1985); Tan et al. "Development and optimization of anti-HIV nucleoside analogs and prodrugs: A review of their cellular pharmacology, structure-activity relationships and pharmacokinetics", Adv. Drug Delivery Rev. 39(1-3): 117-151 (1999); Design of Prodrugs (Bundgaard H. ed.) 1985 Elsevier Science Publishers B. V. (Biomedical Division), Chapter 1; Design of Prodrugs: Bioreversible derivatives for various functional groups and chemical entities (Hans Bundgaard); Bundgaard et al. Int. J. of Pharmaceutics 22 (1984) 45 - 56 (Elsevier); Bundgaard et al. Int. J. of Pharmaceutics 29 (1986) 19 - 28 (Elsevier); Bundgaard et al. J. Med. Chem. 32 (1989) 2503 - 2507 Chem. Abstracts 93, 137935y (Bundgaard *et al*.); Chem. Abstracts 95, 138493f (Bundgaard *et al*.); Chem. Abstracts 95, 138592n (Bundgaard *et al*.); Chem. Abstracts 110, 57664p (Alminger *et al*.); Chem. Abstracts 115, 64029s (Buur *et al*.); Chem. Abstracts 115, 189582y (Hansen *et al*.); Chem. Abstracts 117, 14347q (Bundgaard *et al*.); Chem. Abstracts 117, 55790x (Jensen *et al*.); and Chem. Abstracts 123, 17593b (Thomsen *et al*.)*.*

The terms "administered", "administration", or "administering" a compound will be understood to mean providing any compound of the invention to an individual, including an animal, in need of treatment by bringing such individual in contact with, or otherwise exposing such individual to, such compound.

The term "in vitro" refers to a biological entity, a biological process, or a biological reaction outside the body in artificial conditions. For example a cell grown in vitro is to be understood as a cell grown in an environment outside the body, e.g., in a test tube, a culture tray or a microtiter plate.

The term "therapeutically effective amount" means the amount of the subject compound that will elicit the biological, physiological, pharmacological, therapeutic or medical response of a cell, tissue, system, body, animal, individual, patient or human that is being sought by the researcher, scientist, pharmacologist, pharmacist, veterinarian, medical doctor, or other clinician, e.g., lessening of the effects/symptoms of a disorder or disease, such as a proliferative disorder or disease, for example, a cancer or tumor, or killing or inhibiting growth of a proliferating cell, such as a tumor cell. The therapeutically effective amount can be determined by standard procedures, including those described below in the section "Dosages".

The term "further treated", "further administer", or "further administered" means that different therapeutic agents or compounds may be administered together, alternatively or intermittently. Such further administration may be temporally or spatially separated, for example, at different times, on different days or via different modes or routes of administration.

### Certain compounds of the present invention

One aspect of the invention relates to a compound having a structure represented by formula (I) or any tautomeric or stereoisomeric form thereof, wherein one or more hydrogen atoms in moieties indicated as being "substituted" from R¹, R², R³, R⁴, R⁵, and/or R⁶ may be independently substituted by one or more substituents -[(R^{Sm})ₙ], as described above.

Table 4 shows a selection of exemplary compounds of the present invention having a substituted aryl or heteroaryl as R¹, i.e. a group -R¹-[(R^{Sm})ₙ]ₓ, and how the x substituent(s) -[(R^{Sm})ₙ] can be dissected in each case.

In certain embodiments, R¹ is substituted or unsubstituted phenyl. In certain such embodiments, R¹ is phenyl substituted by 1, 2 or 3 substituents R⁷, wherein each R⁷ is independently selected from the list of: -CN, -NO₂, -N₃, -F, -Cl, -Br, -I, and substituted or unsubstituted: alkyl, alkenyl, alkynyl, cycloalkyl, cycloalkenyl, C-linked heterocycloalkyl, C-linked heterocycloalkenyl, aryl, C-linked heteroaryl, -C(=O)-R^{a}, -C(=O)-OR^{a}, -C(=O)-N(R^{a})₂, -O-R^{a}, -S-R^{a}, -SO-R^{a}, -SO₂-R^{a}, -SO₂-N(R^{a})₂, N-linked heterocycloalkyl, N-linked heteroaryl, -N(R^{a})-R^{a}, -N(R^{a})-OR^{a}, -N(R^{a})-N(R^{a})₂, -N(R^{a})-C(=O)-R^{a}, -N(R^{a})-C(=O)-OR^{a}, -N(R^{a})-C(=O)-N(R^{a})₂, and -N(R^{a})-SO₂-R^{a}. In certain embodiments, R¹ is phenyl substituted by at least one substituent R⁷ in 4-position, and in certain embodiments, R¹ is phenyl substituted by at least one substituent R⁷ in 4-position and a second substituent R⁷ in 2-position. In certain of such embodiments, the substituent R⁷ in 4-position is a substituent -Y-R⁸, wherein Y is selected from the list of -C(=O)NH-, -NH-C(=O)-, -SO₂-NH-, and -NH-SO₂-, and wherein R⁸ is selected from the list of substituted or unsubstituted: alkyl, alkenyl, alkynyl, cycloalkyl, cycloalkenyl, C-linked heterocycloalkyl, C-linked heterocycloalkenyl, aryl, C-linked heteroaryl.

In certain embodiments, R² is substituted or unsubstituted -C₁₋₆-alkyl. In particular embodiments, R² is methyl.

In certain embodiments, R³ and R⁴ are independently selected from hydrogen, and substituted or unsubstituted -C₁₋₁₀-alkyl. In certain embodiments, R³ and R⁴ are different. In certain of such embodiments, one of R³ and R⁴ is hydrogen, and the other is substituted or unsubstituted -C₁₋₆alkyl. In particular embodiments, one of R³ and R⁴ is hydrogen, and the other is ethyl. In certain such embodiments, the compound of formula (I) is present as a racemic mixture. In certain other embodiments, the compound of formula (I) is an isolated enantiomer. In particular such embodiments, the compound has a structure represented by the general formula (Ia), wherein R⁴ is hydrogen. In other particular embodiments, the compound has a structure represented by the general formula (Ia), wherein R³ is hydrogen

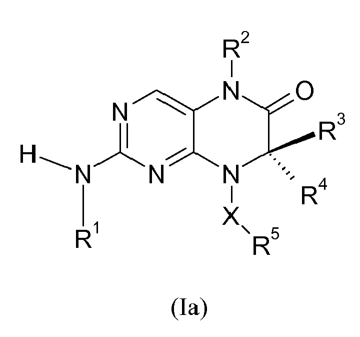

In certain embodiments, X is -O-. In particular such embodiments, R⁵ is selected from hydrogen, and substituted or unsubstituted -C₁₋₆-alkyl. In particular embodiments, R⁵ is methyl.

In certain embodiments, X is -N-R⁶. In particular such embodiments, R⁵ and R⁶, together with the nitrogen atom they are attached to, do not form a substituted or unsubstituted: 4- to 8-membered heterocycloalkyl or heterocycloalkenyl comprising 0, 1 or 2 additional heteroatoms selected from N and O. In particular such embodiments R⁵ and R⁶ are independently selected from hydrogen, and substituted or unsubstituted -C₁₋₆-alkyl. In particular embodiments, R⁵ is methyl, and R⁶ is selected from substituted or unsubstituted -C₁₋₆-alkyl. In particular such embodiments, R⁵ is methyl, and R⁶ is -C₁₋₆-alkyl substituted with a substituent taken from the list of substituted or unsubstituted: -C₂₋₆-alkenyl, -C₃₋₆-cycloalkyl, aryl and heteroaryl.

Indeed, it will be well known to a person of ordinary skill in the art in light of the instant invention, that specificity of compounds of the invention to particular kinase classes or kinases can be changed by modification of the substituents attached to the dihydropteridin-6-one framework (see, for example, the patent applications cited in "Background to the Invention" or McInnes et al. "Strategies for the Design of Potent and Selective Kinase Inhibitors" Current Pharmaceutical Design, Volume 11, Number 14, May 2005, pp. 1845-1863(19)).

Those skilled in the art will recognize that all specific combinations of the individual possible residues of the variable regions of the compounds as disclosed herein, i.e. R¹, R², R³, R⁴, R⁵, R⁶, R⁷, R⁸, X, and Y are within the scope of the invention.

In one embodiment of the present invention, compounds of the invention have a molecular weight of between 245 and 1000, particularly between 400 and 900, more particularly between 450 and 850, and even more particularly between 500 and 800. In certain embodiments, compounds of the invention have one or more of the following characteristics: (i) not more than 5 hydrogen bond donors, (ii) not more than 10 hydrogen bond acceptors, and (iii) not more than 10 rotatable bonds (excluding bonds to terminal atoms). In certain embodiments, the compounds of formula (I) are in accordance with Lipinski's "Rule of Five" (Lipinski, Adv. Drug Del. Rev. 1997; 23: 3), by having a molecular weight below 500, not more than 5 hydrogen bond donors, not more than 10 hydrogen bond acceptors, and a cLogP value between -2 and 5.

In one embodiment of the present invention, compounds of the invention are inhibitors of the activity of Plk1. In certain embodiments, the compounds of the present invention inhibit Plk1 activity with IC₅₀ values below 1 µM, such IC₅₀ value being determined in accordance with the Plk1 inhibition assay described in the examples shown below. In certain embodiments, the IC₅₀ value is below 0.5 µM, below 0.2 µM, below 0.1 µM, or below 0.01 µM.

In one embodiment of the present invention, compounds of the invention have a purity of more than 90%, more than 95%, more than 98%, or more than 99%. Such compounds may exist in one or more crystalline forms, including two or more polymorphic forms, and may exist as dry solids or as solvates including defined amounts of solvents, including hydrates including defined amounts of water.

In another embodiment, compounds of the invention are the planned and deliberate products of a synthetic chemistry scheme, i.e., produced by specific and planned chemical processes conducted in reaction vessels, and not by degradation, metabolism or fermentation, or produced as impurities or by-products in the synthesis of other compounds.

In certain embodiments, compounds of the invention are purified or isolated, e.g., to have a purity of at least 80%, preferably at least 90%, more preferably at least 95%, such as at least 97%, at least 98% or even at least 99%. Purity, as used herein, can refer to either absolute or relative purity. Absolute purity refers to the amount of a compound of the invention obtained as the product of a synthetic chemistry scheme, either before or after one or more purification steps. Relative purity refers to the amount of a compound of the invention relative to one or more impurities such as by-products, degradation products (e.g., metabolites, products of oxidation or hydrolysis, etc.) and/or compounds that degrade to form a compound of the invention (e.g., precursors or prodrugs), e.g., that may be present in the product of a synthetic chemistry scheme. Thus, absolute purity refers to the amount of a compound relative to all others, while relative purity is generally unaffected by the addition of unrelated compounds, such as excipients, stabilizers, or other medicaments for conjoint administration. Purity can be assessed based upon weight, volume or molar ratios of one compound relative to others. Purity can be measured by a variety of analytical techniques, including elemental abundance, UV-visible spectrometry, HPLC, GC-MS, NMR, mass spectrometry, and thin layer chromatography, preferably by HPLC, GC-MS, or NMR.

Yet another aspect of the invention relates to prodrugs of a compound described above.

### Particular embodiments

In a particular aspect, the invention relates to a compound represented by a structure of formula (Ib) 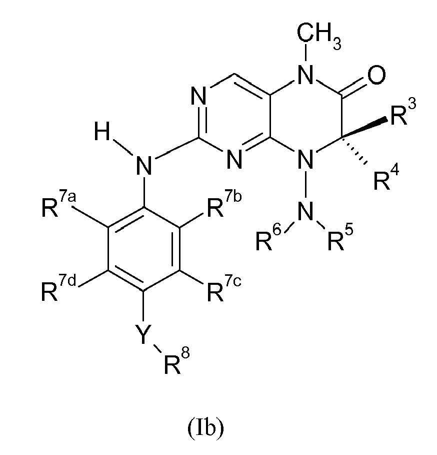 or any tautomeric or stereoisomeric form thereof, wherein
R³ to R⁶, R⁸ and Y are as defined above;
R^{7a} is selected from the list of: -hydrogen, -CN, -F, -Cl, -Br, -I, and substituted or unsubstituted: -C₁₋₆-alkyl, -C₁₋₆-alkenyl, -C₁₋₆-alkynyl, -O-R^{c}, -S-R^{d}, -SO-R^{d}, -SO₂-R^{d}, and - N(R^{c})-R^{c}, wherein R^{c} is selected from the list of: hydrogen and R^{d}, and wherein R^{d} is selected from C₁₋₆-alkyl, C₁₋₆-alkenyl, and C₁₋₆-alkynyl; and
R^{7b}, R^{7c} and R^{7d} are each independently selected from hydrogen, -F, -Cl, -Br, -I, -methyl, -CH₂F, -CHF₂, -CF₃, -O-methyl, -O-CH₂F, -O-CHF₂, and -O-CF₃.

In certain embodiments, Y is -C(=O)-NH-. Such embodiments include, but are not limited to, compounds represented by formula (Ib) where R⁸ is N-methylpiperidin-4-yl.

In certain embodiments, R^{7a} is substituted or unsubstituted -O-C₁₋₃-alkyl. Such embodiments include, but are not limited to, compounds represented by formula (Ib) where R^{7a} is -O-Me.

In certain embodiments, R^{7b}, R^{7c}, and R^{7d} are each hydrogen. In certain embodiments, only one of R^{7b}, R^{7c}, and R^{7d} is different from hydrogen.

In certain embodiments, R³ and R⁴ are independently selected from hydrogen, and substituted or unsubstituted -C₁₋₁₀-alkyl. In certain embodiments, R³ and R⁴ are different. In certain of such embodiments, one of R³ and R⁴ is hydrogen, and the other is substituted or unsubstituted -C₁₋₆-alkyl. In particular embodiments, one of R³ and R⁴ is hydrogen, and the other is ethyl. In certain such embodiments, the compound of formula (Ib) is present as a racemic mixture. In certain other embodiments, the compound of formula (Ib) is an isolated enantiomer. In particular such embodiments, the compound has a structure represented by the general formula (Ib), wherein R⁴ is hydrogen. In further particular embodiments, R³ is ethyl and R⁴ is hydrogen. In particular other such embodiments, the compound has a structure represented by the general formula (Ib), wherein R³ is hydrogen. In further particular embodiments, R⁴ is ethyl and R³ is hydrogen.

In certain embodiments, R⁵ and R⁶ are independently selected from hydrogen, and substituted or unsubstituted -C₁₋₆-alkyl. In particular embodiments, R⁵ is methyl, and R⁶ is selected from substituted or unsubstituted -C₁₋₆-alkyl. In particular such embodiments, R⁵ is methyl, and R⁶ is -C₁₋₆-alkyl substituted with a substituent taken from the list of: -C₂₋₆-alkenyl, - C₃₋₆-cycloalkyl, aryl and heteroaryl.

In certain embodiments of Formula I, the compound is selected from:
4-(8-Dimethylamino-7-ethyl-5-methyl-6-oxo-5,6,7,8-tetrahydro-pteridin-2-ylamino)-3-methoxy-N-(1-methyl-piperidin-4-yl)-benzamide;
4-(8-Dimethylamino-7-ethyl-5-methyl-6-oxo-5,6,7,8-tetrahydro-pteridin-2-ylamino)-3-methoxy-N-(2-pyrrolidin-1-yl-ethyl)-benzamide;
4-(8-Dimethylamino-7-ethyl-5-methyl-6-oxo-5,6,7,8-tetrahydro-pteridin-2-ylamino)-N-(1-ethyl-piperidin-4-ylmethyl)-3-methoxy-benzamide;
4-(8-Dimethylamino-7-ethyl-5-methyl-6-oxo-5,6,7,8-tetrahydro-pteridin-2-ylamino)-N-(1-isopropyl-piperidin-4-yl)-3-methoxy-benzamide;
N-(2-Diethylamino-ethyl)-4-(8-dimethylamino-7-ethyl-5-methyl-6-oxo-5,6,7,8-tetrahydro-pteridin-2-ylamino)-3-methoxy-benzamide;
4-(8-Dimethylamino-7-ethyl-5-methyl-6-oxo-5,6,7,8-tetrahydro-pteridin-2-ylamino)-3-methoxy-N-(2-piperidin-1-yl-ethyl)-benzamide;
N-(2-Diisopropylamino-ethyl)-4-(8-dimethylamino-7-ethyl-5-methyl-6-oxo-5,6,7,8-tetrahydro-pteridin-2-ylamino)-3-methoxy-benzamide;
4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-phenyl]-benzamide;
4-(8-Dimethylamino-7-ethyl-5-methyl-6-oxo-5,6,7,8-tetrahydro-pteridin-2-ylamino)-N-[4-(4-ethyl-piperazin-1-yl)-phenyl]-3-methoxy-benzamide;
4-(8-Dimethylamino-7-ethyl-5-methyl-6-oxo-5,6,7,8-tetrahydro-pteridin-2-ylamino)-3-methoxy-N-(4-piperidin-1-ylmethyl-phenyl)-benzamide;
4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-benzyl]-benzamide;
N-(1-Benzyl-piperidin-4-ylmethyl)-4-(8-dimethylamino-7-ethyl-5-methyl-6-oxo-5,6,7,8-tetrahydro-pteridin-2-ylamino)-3-methoxy-benzamide;
N-[trans-4-(4-Cyclopropylmethyl-piperazin-1-yl)-cyclohexyl]-4-(8-dimethylamino-7-ethyl-5-methyl-6-oxo-5,6,7,8-tetrahydro-pteridin-2-ylamino)-3-methoxy-benzamide;
N-(1'-Benzyl-[1,4']bipiperidinyl-4-yl)-4-(8-dimethylamino-7-ethyl-5-methyl-6-oxo-5,6,7,8-tetrahydro-pteridin-2-ylamino)-3-methoxy-benzamide;
4-[8-(Benzyl-methyl-amino)-7-ethyl-5-methyl-6-oxo-5,6,7,8-tetrahydro-pteridin-2-ylamino]-3-methoxy-N-(1-methyl-piperidin-4-yl)-benzamide;
4-[8-(Benzyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)-cyclohexyl]-3-methoxy-benzamide;
4-[8-(Cyclopropylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide;
4-[8-(Cyclopropylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide;
N-[trans-4-(4-Cyclopropylmethyl-piperazin-1-yl)-cyclohexyl]-4-[7-ethyl-5-methyl-8-(methyl-propyl-amino)-6-oxo-5,6,7,8-tetrahydro-pteridin-2-ylamino]-3-methoxy-benzamide;
4-[7-Ethyl-5-methyl-8-(methyl-propyl-amino)-6-oxo-5,6,7,8-tetrahydro-pteridin-2-ylamino]-3-methoxy-N-(1-methyl-piperidin-4-yl)-benzamide;
4-[8-(Benzyl-methyl-amino)-7-ethyl-5-methyl-6-oxo-5,6,7,8-tetrahydro-pteridin-2-ylamino]-N-(1-ethyl-piperidin-4-ylmethyl)-3-methoxy-benzamide;
4-[7-Ethyl-8-(isobutyl-methyl-amino)-5-methyl-6-oxo-5,6,7,8-tetrahydro-pteridin-2-ylamino]-3-methoxy-N-(1-methyl-piperidin-4-yl)-benzamide;
4-[7-Ethyl-8-(isobutyl-methyl-amino)-5-methyl-6-oxo-5,6,7,8-tetrahydro-pteridin-2-ylamino]-N-(1-ethyl-piperidin-4-ylmethyl)-3-methoxy-benzamide;
N-[trans-4-(4-Cyclopropylmethyl-piperazin-1-yl)-cyclohexyl]-4-[7-ethyl-8-(isobutylmethyl-amino)-5-methyl-6-oxo-5,6,7,8-tetrahydro-pteridin-2-ylamino]-3-methoxy-benzamide;
4-[7-Ethyl-8-(ethyl-methyl-amino)-5-methyl-6-oxo-5,6,7,8-tetrahydro-pteridin-2-ylamino]-3-methoxy-N-(1-methyl-piperidin-4-yl)-benzamide;
N-[trans-4-(4-Cyclopropylmethyl-piperazin-1-yl)-cyclohexyl]-4-[7-ethyl-8-(ethylmethyl-amino)-5-methyl-6-oxo-5,6,7,8-tetrahydro-pteridin-2-ylamino]-3-methoxy-benzamide;
4-[8-(Cyclopentylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide;
4-[8-(Cyclopentylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide;
4-[8-(Cyclopentylmethyl-methyl-amino)-7-ethyl-5-methyl-6-oxo-5,6,7,8-tetrahydropteridin-2-ylamino]-N-(1-ethyl-piperidin-4-ylmethyl)-3-methoxy-benzamide;
4-(7-Ethyl-5-methyl-8-methylamino-6-oxo-5,6,7,8-tetrahydro-pteridin-2-ylamino)-3-methoxy-N-(1-methyl-piperidin-4-yl)-benzamide;
N-[trans-4-(4-Cyclopropylmethyl-piperazin-1-yl)-cyclohexyl]-4-(7-ethyl-5-methyl-8-methylamino-6-oxo-5,6,7,8-tetrahydro-pteridin-2-ylamino)-3-methoxy-benzamide;
4-(7-Ethyl-5-methyl-8-methylamino-6-oxo-5,6,7,8-tetrahydro-pteridin-2-ylamino)-N-(1-ethyl-piperidin-4-ylmethyl)-3-methoxy-benzamide;
4-[8-(Cyclobutylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide
4-[8-(Cyclobutylmethyl-methyl-amino)-7-ethyl-5-methyl-6-oxo-5,6,7,8-tetrahydropteridin-2-ylamino]-N-(1-ethyl-piperidin-4-ylmethyl)-3-methoxy-benzamide;
4-[8-(Cyclobutylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide;
   where such compounds have the structures as disclosed in Table 2 below. In case of a discrepancy between the chemical name given above and the corresponding structure shown in Table 1, the structure should be regarded as correct, and the name amended accordingly.

In certain such embodiments, a compound listed above is present as a racemic mixture. In certain other embodiments, a compound listed above is an isolated enantiomer. In particular such embodiments, a compound is the (R)-enantiomer. In particular other embodiments, a compound is the (S)-enantiomer. In one embodiment of an isolated enantiomer, R³ is hydrogen. In another such embodiment, R⁴ is hydrogen.

### Formulations, Dosages and Applications

The present invention further provides a pharmaceutical composition including a compound as described above, or prodrug thereof, and a pharmaceutically acceptable diluent, excipient or carrier, including pharmaceutical compositions including a therapeutically effective amount of such compound or prodrug.

### Formulations

The compositions of this invention can be formulated and administered to treat individuals in need by any means that produces contact of the active ingredient with the agent's site of action, such as a cell, in the body of an individual. They can be administered by any conventional means available for use in conjunction with pharmaceuticals, either as individual therapeutic active ingredients or in a combination of therapeutic active ingredients. They can be administered alone, but are generally administered with a pharmaceutically acceptable diluent, excipient or carrier selected on the basis of the chosen route of administration and standard pharmaceutical practice.

A pharmaceutical composition comprising less than a therapeutically effective amount of any of the compounds described above, or a prodrug thereof, may also be used, such as when used in combination with another pharmaceutical composition, such as an anti-cancer agent, so that such combination is therapeutically effective, or may be useful for prophylactic treatment.

Pharmaceutical compositions for use in accordance with the present invention may be formulated in conventional manner using one or more pharmaceutically acceptable diluents, excipients or carriers. The pharmaceutical compositions of the invention can be formulated for a variety of routes of administration, including systemic and topical or localized administration. Techniques and formulations generally may be found in Remington's Pharmaceutical Sciences, Meade Publishing Co., Easton, PA. As described in detail below, the pharmaceutical compositions of the present invention may be specially formulated for administration in solid or liquid form, including those adapted for the following: (1) oral administration, for example, drenches (aqueous or non-aqueous solutions or suspensions), tablets, capsules, boluses, powders, granules, pastes for application to the tongue; (2) parenteral administration, for example, by subcutaneous, intramuscular or intravenous injection as, for example, a sterile solution or suspension; (3) topical application, for example, as a cream, ointment or spray applied to the skin; or (4) intravaginally or intrarectally, for example, as a pessary, cream or foam. In certain embodiments, the pharmaceutical preparations may be non-pyrogenic, i.e., do not substantially elevate the body temperature of a patient.

Wetting agents, emulsifiers and lubricants, such as sodium lauryl sulfate and magnesium stearate, as well as coloring agents, release agents, coating agents, sweetening, flavoring and perfuming agents, preservatives and antioxidants can also be present in the compositions.

Examples of pharmaceutically acceptable antioxidants include: (1) water soluble antioxidants, such as ascorbic acid, cysteine hydrochloride, sodium bisulfate, sodium metabisulfite, sodium sulfite and the like; (2) oil-soluble antioxidants, such as ascorbyl palmitate, butylated hydroxyanisole (BHA), butylated hydroxytoluene (BHT), lecithin, propyl gallate, alpha-tocopherol, and the like; and (3) metal chelating agents, such as citric acid, ethylenediamine tetraacetic acid (EDTA), sorbitol, tartaric acid, phosphoric acid, and the like.

Formulations of the present invention include those suitable for oral, nasal, topical (including buccal and sublingual), rectal, vaginal and/or parenteral administration. The formulations may conveniently be presented in unit dosage form and may be prepared by any methods well known in the art of pharmacy. The amount of active ingredient which can be combined with a carrier material to produce a single dosage form will vary depending upon the host being treated, as well as the particular mode of administration. The amount of active ingredient which can be combined with a carrier material to produce a single dosage form will generally be that amount of inhibitor which produces a therapeutic effect. Generally, out of one hundred percent, this amount will range from about 1 percent to about ninety-nine percent of active ingredient, preferably from about 5 percent to about 70 percent, most preferably from about 10 percent to about 30 percent.

Methods of preparing these formulations or compositions include the step of bringing into association a compound of the present invention with the carrier and, optionally, one or more accessory ingredients. In general, the formulations are prepared by uniformly and intimately bringing into association a compound of the present invention with liquid carriers, or finely divided solid carriers, or both, and then, if necessary, shaping the product.

For systemic administration, injection is preferred, including intramuscular, intravenous, intraperitoneal, and subcutaneous (i.m., i.v., i.p., and s.c. respectively). The phrases "systemic administration", "administered systemically", "peripheral administration", and "administered peripherally" as used herein mean the administration of a compound, drug or other material other than directly into the central nervous system, such that it enters the patient's system and, thus, is subject to metabolism and other like processes, for example, subcutaneous administration.

For injection, the pharmaceutical compositions of the invention can be formulated in liquid solutions, preferably in physiologically compatible buffers such as Hank's solution or Ringer's solution. In addition, the pharmaceutical compositions may be formulated in solid form and redissolved or suspended immediately prior to use. Lyophilized forms are also included.

Pharmaceutical compositions of the invention may be formulated to be suitable for oral administration may be in the form of capsules, cachets, sachets, pills, tablets, lozenges (using a flavored basis, usually sucrose and acacia or tragacanth), powders, granules, or as a solution or a suspension in an aqueous or non-aqueous liquid, or as an oil-in-water or water-in-oil liquid emulsion, or as an elixir or syrup, or as pastilles (using an inert base, such as gelatin and glycerin, or sucrose and acacia) and/or as mouth washes and the like, each containing a predetermined amount of a compound of the present invention as an active ingredient. A compound of the present invention may also be administered as a bolus, electuary or paste.

In formulating the pharmaceutical compositions of the invention in solid dosage forms for oral (p.o.) administration (capsules, tablets, pills, dragees, powders, granules and the like), a compound of the invention as active ingredient is mixed with one or more pharmaceutically acceptable carriers, such as sodium citrate or dicalcium phosphate, and/or any of the following:
(1) fillers or extenders, such as starches, lactose, sucrose, glucose, mannitol, and/or silicic acid;
(2) binders, such as, for example, carboxymethylcellulose, alginates, gelatin, polyvinyl pyrrolidone, sucrose and/or acacia; (3) humectants, such as glycerol; (4) disintegrating agents, such as agar-agar, calcium carbonate, potato or tapioca starch, alginic acid, certain silicates, and sodium carbonate; (5) solution retarding agents, such as paraffin; (6) absorption accelerators, such as quaternary ammonium compounds; (7) wetting agents, such as, for example, cetyl alcohol and glycerol monostearate; (8) absorbents, such as kaolin and bentonite clay; (9) lubricants, such a talc, calcium stearate, magnesium stearate, solid polyethylene glycols, sodium lauryl sulfate, and mixtures thereof; and (10) coloring agents. In the case of capsules, tablets and pills, the pharmaceutical compositions may also comprise buffering agents. Solid compositions of a similar type may also be employed as fillers in soft and hard-filled gelatin capsules using such excipients as lactose or milk sugars, high molecular weight polyethylene glycols, and the like.

Gelatin capsules contain a compound of the present invention as active ingredient and powdered carriers, such as lactose, starch, cellulose derivatives, magnesium stearate, stearic acid, and the like. Similar carriers can be used to make compressed tablets. Both tablets and capsules can be manufactured as sustained release products to provide for continuous release of medication over a period of hours. Compressed tablets can be sugar-coated or film-coated to mask any unpleasant taste and protect the tablet from the atmosphere, or enteric coated for selective disintegration in the gastrointestinal tract. Solid compositions of a similar type are also employed as fillers in soft and hard-filled gelatin capsules; preferred materials in this connection also include lactose or milk sugar as well as high molecular weight polyethylene glycols. A preferred formulation is a solution or suspension in an oil, for example olive oil, Miglyol, or Capmul, in a soft gelatin capsule. Antioxidants may be added to prevent long-term degradation as appropriate.

A tablet may be made by compression or molding, optionally with one or more accessory ingredients. Compressed tablets may be prepared using a binder (for example, gelatin or hydroxypropylmethyl cellulose), lubricant, inert diluent, preservative, disintegrant (for example, sodium starch glycolate or cross-linked sodium carboxymethyl cellulose), surface-active or dispersing agent. Molded tablets may be made by molding in a suitable machine a mixture of the powdered inhibitor moistened with an inert liquid diluent.

The tablets and other solid dosage forms of the pharmaceutical compositions of the present invention, such as dragees, capsules, pills and granules, may optionally be scored or prepared with coatings and shells, such as enteric coatings and other coatings well known in the pharmaceutical-formulating art. They may also be formulations so as to provide slow or controlled release of the active ingredient therein using, for example, hydroxypropylmethyl cellulose in varying proportions to provide the desired release profile, other polymer matrices, liposomes and/or microspheres. They may be sterilized by, for example, filtration through a bacteria-retaining filter, or by incorporating sterilizing agents in the form of sterile solid compositions which can be dissolved in sterile water, or some other sterile injectable medium immediately before use. These compositions may also optionally contain opacifying agents and may be of a composition that they release the active ingredient(s) only, or preferentially, in a certain portion of the gastrointestinal tract, optionally in a delayed manner. Examples of embedding compositions which can be used include polymeric substances and waxes. The active ingredient can also be in micro-encapsulated form, if appropriate, with one or more of the above-described excipients.

Liquid dosage forms for oral administration of the pharmaceutical compositions of the invention include pharmaceutically acceptable emulsions, microemulsions, solutions, suspensions, syrups, and elixirs. In addition to the active ingredient, the liquid dosage forms may contain inert diluents commonly used in the art, such as, for example, water or other solvents, solubilizing agents and emulsifiers, such as ethyl alcohol, isopropyl alcohol, ethyl carbonate, ethyl acetate, benzyl alcohol, benzyl benzoate, propylene glycol, 1,3-butylene glycol, oils (in particular, cottonseed, groundnut, corn, germ, olive, castor and sesame oils), glycerol, tetrahydrofuryl alcohol, polyethylene glycols and fatty acid esters of sorbitan, and mixtures thereof.

Besides inert diluents, the pharmaceutical compositions for oral administration can also include adjuvants such as wetting agents, emulsifying and suspending agents, sweetening, flavoring, coloring, perfuming, and preservative agents.

Suspensions, in addition to the pharmaceutical composition of the present invention, may contain suspending agents as, for example, ethoxylated isostearyl alcohols, polyoxyethylene sorbitol and sorbitan esters, microcrystalline cellulose, aluminum metahydroxide, bentonite, agar-agar, and tragacanth, and mixtures thereof.

For buccal administration the pharmaceutical compositions may take the form of tablets or lozenges formulated in a conventional manner.

For administration by inhalation, the pharmaceutical compositions of the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of, for example, gelatin for use in an inhaler or insufflator may be formulated containing a powder mix of the therapeutic agents and a suitable powder base such as lactose or starch.

The pharmaceutical compositions may be formulated for parenteral administration by injection, e.g., by bolus injection or continuous infusion. Formulations for injection may be presented in unit dosage form, e.g., in ampoules or in multi-dose containers, with an added preservative. The pharmaceutical compositions may take such forms as suspensions, solutions or emulsions in oily or aqueous vehicles, and may contain formulatory agents such as suspending, stabilizing and/or dispersing agents. Alternatively, the active ingredient may be in powder form for constitution with a suitable vehicle, e.g., sterile pyrogen-free water, before use.

The phrases "parenteral administration" and "administered parenterally" as used herein means modes of administration other than enteral and topical administration, usually by injection, and includes, without limitation, intravenous, intramuscular, intraarterial, intrathecal, intracapsular, intraorbital, intracardiac, intradermal, intraperitoneal, transtracheal, subcutaneous, subcuticular, intraarticular, subcapsular, subarachnoid, intraspinal and intrasternal injection and infusion.

Pharmaceutical compositions of this invention suitable for parenteral administration comprise one or more inhibitors of the invention in combination with one or more pharmaceutically acceptable sterile isotonic aqueous or nonaqueous solutions, dispersions, suspensions or emulsions, or sterile powders which may be reconstituted into sterile injectable solutions or dispersions just prior to use, which may contain antioxidants, buffers, bacteriostats, solutes which render the formulation isotonic with the blood of the intended recipient or suspending or thickening agents.

Examples of suitable aqueous and nonaqueous carriers which may be employed in the pharmaceutical compositions of the invention include water, ethanol, polyols (such as glycerol, propylene glycol, polyethylene glycol, and the like), and suitable mixtures thereof, vegetable oils, such as olive oil, and injectable organic esters, such as ethyl oleate. Proper fluidity can be maintained, for example, by the use of coating materials, such as lecithin, by the maintenance of the required particle size in the case of dispersions, and by the use of surfactants.

These pharmaceutical compositions may also contain adjuvants such as preservatives, wetting agents, emulsifying agents and dispersing agents. Prevention of the action of microorganisms may be ensured by the inclusion of various antibacterial and antifungal agents, for example, paraben, chlorobutanol, phenol sorbic acid, and the like. It may also be desirable to include isotonic agents, such as sugars, sodium chloride, and the like into the pharmaceutical compositions. In addition, prolonged absorption of the injectable pharmaceutical form may be brought about by the inclusion of agents that delay absorption such as aluminum monostearate and/or gelatin.

In addition to the formulations described previously, the pharmaceutical compositions may also be formulated as a depot preparation. Such long acting formulations may be administered by implantation (for example subcutaneously or intramuscularly) or by intramuscular injection. Thus, for example, the pharmaceutical compositions may be formulated with suitable polymeric or hydrophobic materials (for example as an emulsion in an acceptable oil) or ion exchange resins, or as sparingly soluble derivatives, for example, as a sparingly soluble salt.

Systemic administration can also be by transmucosal or transdermal means. For transmucosal or transdermal administration, penetrants appropriate to the barrier to be permeated are used in the formulation. Such penetrants are generally known in the art, and include, for example, for transmucosal administration bile salts and fusidic acid derivatives. In addition, detergents may be used to facilitate permeation. Transmucosal administration may be through nasal sprays or using suppositories. For topical administration, the pharmaceutical compositions of the invention are formulated into ointments, salves, gels, or creams as generally known in the art. A wash solution can be used locally to treat an injury or inflammation to accelerate healing.

In some cases, in order to prolong the therapeutic effect of an inhibitor, it is desirable to slow the absorption of the inhibitor from subcutaneous or intramuscular injection. This may be accomplished by the use of a liquid suspension of crystalline or amorphous material having poor water solubility. The rate of absorption of the inhibitor then depends upon its rate of dissolution which, in turn, may depend upon crystal size and crystalline form. Alternatively, delayed absorption of a parenterally administered inhibitor form is accomplished by dissolving or suspending the inhibitor in an oil vehicle.

Pharmaceutical compositions of the invention may be formulated for rectal or vaginal administration as a suppository, which may be prepared by mixing one or more compounds of the invention with one or more suitable nonirritating excipients or carriers comprising, for example, cocoa butter, polyethylene glycol, a suppository wax or a salicylate, and which is solid at room temperature, but liquid at body temperature and, therefore, will melt in the rectum or vaginal cavity and release the active inhibitor.

Formulations of the pharmaceutical compositions of the present invention, which are suitable for vaginal administration, also include pessaries, tampons, creams, gels, pastes, foams or spray formulations containing such carriers as are known in the art to be appropriate.

Dosage forms for the topical or transdermal administration of a compound of this invention include powders, sprays, ointments, pastes, creams, lotions, gels, solutions, patches and inhalants. Such compound may be mixed under sterile conditions with a pharmaceutically acceptable carrier, and with any preservatives, buffers, or propellants which may be required.

The ointments, pastes, creams and gels may contain, in addition to a compound of the invention, excipients, such as animal and vegetable fats, oils, waxes, paraffins, starch, tragacanth, cellulose derivatives, polyethylene glycols, silicones, bentonites, silicic acid, talc and zinc oxide, or mixtures thereof.

Powders and sprays can contain, in addition to a compound of this invention, excipients such as lactose, talc, silicic acid, aluminum hydroxide, calcium silicates and polyamide powder, or mixtures of these substances. Sprays can additionally contain customary propellants, such as chlorofluorohydrocarbons and volatile unsubstituted hydrocarbons, such as butane and propane.

Transdermal patches have the added advantage of providing controlled delivery of a compound of the present invention to the body. Such dosage forms can be made by dissolving or dispersing an inhibitor of the present invention in the proper medium. Absorption enhancers can also be used to increase the flux of the drug across the skin. The rate of such flux can be controlled by either providing a rate controlling membrane or dispersing the compound of the present invention in a polymer matrix or gel.

Ophthalmic formulations, eye ointments, powders, solutions and the like, are also contemplated as being within the scope of this invention.

The pharmaceutical compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. In other embodiments, the pack or dispenser may be further packaged in an outer carton.

A pharmaceutical composition of the present invention can also be formulated as a sustained and/or timed release formulation. Such sustained and/or timed release formulations may be made by sustained release means or delivery devices that are well known to those of ordinary skill in the art, such as those described in U.S. Patent Nos.: 3,845,770; 3,916,899; 3,536,809; 3,598,123; 4,008,719; 4,710,384; 5,674,533; 5,059,595; 5,591,767; 5,120,548; 5,073,543; 5,639,476; 5,354,556; and 5,733,566, the disclosures of which are each incorporated herein by reference. The pharmaceutical compositions of the present invention can be used to provide slow or sustained release of one or more of the active ingredients using, for example, hydroxypropylmethyl cellulose, other polymer matrices, gels, permeable membranes, osmotic systems, multilayer coatings, microparticles, liposomes, microspheres, or the like, or a combination thereof to provide the desired release profile in varying proportions. Suitable sustained release formulations known to those of ordinary skill in the art, including those described herein, may be readily selected for use with the pharmaceutical compositions of the invention. Thus, single unit dosage forms suitable for oral administration, such as, but not limited to, tablets, capsules, gelcaps, caplets, powders, and the like, that are adapted for sustained release are encompassed by the present invention.

Injectable depot forms are made by forming microencapsuled matrices of the subject inhibitors in biodegradable polymers such as polylactide-polyglycolide. Depending on the ratio of drug to polymer, and the nature of the particular polymer employed, the rate of drug release can be controlled. Examples of other biodegradable polymers include poly(orthoesters) and poly(anhydrides). Depot injectable formulations are also prepared by entrapping the drug in liposomes or microemulsions that are compatible with body tissue.

When the compounds of the present invention are administered as pharmaceuticals, to individuals, such as humans and animals, they can be given per se or as a pharmaceutical composition containing, for example, 0.1 to 99.5% (in certain embodiments, 0.5 to 90%) of active ingredient in combination with a pharmaceutically acceptable carrier.

The present invention provides new methods of treating proliferative, degenerative and other disorders or diseases, including cancer, by administering an amount such as a therapeutically effective amount of at least one of the compounds disclosed herein or a prodrug, tautomeric, pharmaceutically acceptable salt, N-oxide or stereoisomeric form thereof. The present invention further provides methods of treating proliferative, degenerative or other disorders or diseases, including cancer, by administering a therapeutically effective combination of at least one of these compounds and another anti-cancer or anti-proliferative agent.

A compound of the present invention may be administered as a salt or prodrug that, upon administration to the individual, is capable of providing directly or indirectly the parent compound, such as a compound as defined herein, or that exhibits activity itself. Nonlimiting examples include a pharmaceutically acceptable salt, alternatively referred to as a "physiologically acceptable salt". In addition, modifications made to a compound can affect its biological activity, in some cases increasing the activity over the parent compound. This activity can be assessed by preparing a salt or prodrug form of the compound, and testing its activity by using methods described herein or other methods known to those of skill in the art.

As will be apparent to a person skilled in the art, through the use of a prodrug of a given subject compound, an individual such as an animal administered or treated with such prodrug will be exposed to, and hence indirectly administered with, the subject compound. Such a procedure may expose those cells associated with a disease, such as a proliferative disease or disorder including cancer, to the subject compound.

The compounds of the present invention may contain an asymmetrically substituted carbon atom, and may be isolated in optically active or racemic forms. It is well known in the art how to prepare optically active forms, such as by resolution of racemic forms or by synthesis from optically active starting materials. All chiral, diastereomeric, racemic forms and all geometric isomeric forms of a structure are intended, unless the specific stereochemistry or isomer form is specifically indicated. All processes used to prepare compounds of the present invention and intermediates made therein are considered to be part of the present invention.

### Dosages

A dosage administered that will be a therapeutically effective amount of the compound sufficient, or reasonably expected by a health-care professional such as a physician, pharmacist or nurse, to result in amelioration of symptoms of, for example, the cancer or tumor will, of course, vary depending upon known factors such as the pharmacodynamic characteristics of the particular active ingredient and its mode and route of administration; age, sex, health and weight of the recipient; nature and extent of symptoms; kind of concurrent treatment, frequency of treatment and the effect desired.

The subject compounds may also be administered in prophylactic treatment. If the compound is administered prior to clinical manifestation of the unwanted condition (e.g., disease or other unwanted state of the host animal) then the treatment is prophylactic (i.e., it protects the individual against initiating, developing or further developing the unwanted condition). The subject compounds may also be administered to prevent a condition, disorder or diseases, such as cancer, or a syndrome complex, such as heart failure or any other medical condition. This includes administration of a compound the intent of which is to reduce the frequency of, or delay the onset of, symptoms of a medical condition in an individual relative to an individual which does not receive the compound. Thus, prevention of cancer includes, for example, reducing the number of detectable cancerous growths, tumors, or malignancies in a population of patients receiving a prophylactic treatment relative to an untreated control population, delaying the appearance of detectable cancerous growths in a treated population versus an untreated control population, and/or delaying disease progression and/or improving the quality of patient life, e.g., by a statistically and/or clinically significant amount.

Toxicity and therapeutic efficacy of pharmaceutical compositions of the present invention can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio LD₅₀/ED₅₀. Therapeutic agents that exhibit large therapeutic indices are useful for many circumstances. In certain circumstances, even therapeutic compositions that appear to exhibit debilitating or toxic side effects may be used, including circumstances where care is taken to design a delivery system that targets such therapeutic agents to the site of affected tissue in order to minimize potential damage to unaffected cells and, thereby, reduce or localize side effects.

The data obtained from cell culture assays and animal studies can be used in formulating a range of dosage for use in humans. The dosage lies preferably within a range of circulating concentrations that include the ED50 with little or no toxicity. The dosage may vary within this range depending upon the dosage form employed and the route of administration utilized. For any agents used in the method of the invention, the therapeutically effective dose can be estimated initially from cell culture assays. A dose may be formulated in animal models to achieve a circulating plasma concentration range that includes the IC₅₀ (i.e., the concentration of the test therapeutic agent which achieves a half-maximal inhibition of symptoms or inhibition of biochemical activity) as determined in cell culture. Such information can be used to more accurately determine useful doses in humans. Levels in plasma may be measured, for example, by high performance liquid chromatography.

It is understood that appropriate doses of therapeutic agents depends upon a number of factors known to those or ordinary skill in the art, e.g., a physician. The dose(s) of the subject compounds will vary, for example, depending upon the identity, size, and condition of the subject or sample being treated, further depending upon the route by which the composition is to be administered, if applicable, and the effect which the practitioner desires the therapeutic to have upon the therapeutic target of targets, such as cells, nucleic acid or polypeptides, through with the disease causes, symptoms or effects are mediated.

Exemplary doses include milligram or microgram amounts of the compounds of the present invention per kilogram of subject or sample weight, e.g., about 1 microgram per kilogram to about 500 milligrams per kilogram, about 100 micrograms per kilogram to about 50 milligrams per kilogram, or about 1 milligram per kilogram to about 5 milligrams per kilogram.

A person skilled in the art will appreciate that doses can also be calculated on a body surface basis. A person of 70 kg has an approximate body surface area of 1.8 square meter, and doses can be expressed as milligram or microgram amounts of the compound per body surface area of subject or sample, e.g. about 50 microgram per square meter to about 15 grams per square meter, about 5 milligrams per square meter to about 1.5 grams per square meter, or about 50 milligram per square meter to about 150 milligrams per square meter.

### Application

The present invention further provides the compounds as described above for therapy. In other aspects, the invention provides the compounds of the present invention for prophylatic uses.

In certain embodiments, said therapy or prophylactic use is the treatment or prevention of a proliferative disorder or disease, such as a tumor or cancer. In certain embodiments, said treatment is the treatment of a cancer that can be treated by the inhibition of the activity of a protein kinase or mutant thereof, such as the inhibition of the activity of Plk1.

Thus, the present invention additionally provides a method for treating an individual, such as a mammal, having a disease-state selected from the group of proliferative disorders or diseases, or inflammatory disorders or diseases, comprising administering to said individual a therapeutically effective amount of a compound, a prodrug, or a pharmaceutical composition of the invention as described above. In certain embodiments, said individual is a human. In certain embodiments, said proliferative disorder or disease is cancer. In certain embodiments, said treatment is the treatment of a cancer that can be treated by the inhibition of the activity of a protein kinase or mutant thereof, such as the inhibition of the activity of Plk1.

The present invention also provides a method for prophylactic treatment of an individual such as an animal, including a mammal, particularly a human, the intent of which is to reduce the frequency of, delay the onset of, or the symptoms of a medical condition, such as cancer, in a subject relative to a subject which does not receive the composition.

In a further aspect, the invention provides methods of treating or preventing an individual suffering from a disease, such as a mammal, including a domestic mammal, cat, dog, horse, sheep, cow, rodent, and human, comprising the step of exposing said individual to an amount, including a therapeutically effective amount, of a subject compound. In certain embodiments, the disease is a proliferative disorder or disease, such as a cancer or tumour. In yet another embodiment, cells associated with said proliferative disorder or disease, including tumour cells included in a cancer, are exposed to the subject compound. In certain embodiments, said compound, or a prodrug thereof, is administered to said individual. In certain embodiments, said treatment is the treatment of a cancer that can be treated by the inhibition of the activity of a protein kinase or mutant thereof, such as the inhibition of the activity of Plk1. In certain embodiments, the disease is an inflammatory disorder or disease. In yet another embodiment, cells associated with said inflammatory disorder or disease are exposed to the subject compound. In certain embodiments, said compound, or a prodrug thereof, is administered to said individual.

In a further aspect, the invention provides a method of killing or inhibiting proliferation or growth of a cell, comprising contacting the cell with a compound of the invention. In one embodiment, the cell is cultured in-vitro, while in an alternative embodiment the cell is present in an individual. In a particular embodiment the cell is a cancer cell, for example a cell from a tumour cell line or a cell included in a tumour, including cancer cells from a tumour that can be treated by the inhibition of the activity of a protein kinase or mutant thereof, such as the inhibition of the activity of Plk1.

Yet another aspect of the invention relates to the use of a compound as described above, or a prodrug thereof, for the preparation of a medicament for the treatment or prevention of a proliferative disorder or disease, including cancer, including cancers that can be treated by the inhibition of the activity of a protein kinase or mutant thereof, such as the inhibition of the activity of Plk1. Additionally, the invention relates to a pharmaceutical composition comprising a compound as described above, or a prodrug thereof, and a pharmaceutically acceptable diluent, excipient or carrier, for the treatment of a proliferative disorder or disease, including cancer, including cancers that can be treated by the inhibition of the activity of a protein kinase or mutant thereof, such as the inhibition of the activity of Plk1.

The subject compounds are useful to treat various disorders or diseases, including proliferative disorders or diseases. The term "proliferative disorder or disease" is also art recognized and includes a disorder or disease affecting an individual, such as an animal, in a manner which is marked by aberrant, or otherwise unwanted, proliferation of a subset of cells of an individual. Cancer and tumors are proliferative disorders or diseases. Cells comprising or derived from a tumor will generally be understood to be a proliferating cell, typically a hyperproliferating cell, and in other circumstances, a tumor cell may be dysplastic, or may have proliferated. In certain embodiments, said treatment is the treatment of a cancer that can be treated by the inhibition of the activity of a protein kinase or mutant thereof, such as the inhibition of the activity of Plk1.

It will be apparent to a person skilled in the art, on reading the disclosure of the instant invention, that the methods, pharmaceutical compositions and packaged pharmaceuticals comprising the subject compounds will be useful for the treatment of other proliferative disorders or diseases, or for killing or inhibiting proliferating cells including tumor cells.

Compounds of the present invention may be useful in the treatment of disease processes which feature abnormal cellular proliferation, such as hyperproliferative diseases, including cancer, benign prostate hyperplasia, familial adenomatosis polyposis, neurofibromatosis, psoriasis, fungal infections, endotoxic shock, hypertrophic scar formation, inflammatory bowel disease, transplant rejection, vascular smooth muscle cell proliferation associated with atherosclerosis, psoriasis, pulmonary fibrosis, arthritis, glomerulonephritis, restenosis following angioplasty or vascular surgery, and other post-surgical stenosis and restenosis. See, for example, U.S. Patent Nos. 6,114,365 and 6,107,305.

The compounds disclosed herein are expected to be useful in the therapy of proliferative or hyperproliferative disorders or diseases such as cancer, autoimmune diseases, viral diseases, fungal diseases, neurodegenerative disorders and cardiovascular disease.

In certain embodiments, tumors may be solid tumors, which are cancer of body tissues other than blood, bone marrow, or the lymphatic system. In other embodiments, tumors may be hematological tumors, such as leukemia and lymphomas. Leukemia is a collective term for malignant diseases characterized by a proliferation of malignantly changed white blood cells. Diseases arising from lymphatic tissue are called lymphomas.

Solid tumors may be selected from: liver cancer, stomach cancer, colon cancer, breast cancer, pancreas cancer, prostate cancer, skin cancer, renal cancer, bone cancer, thyroid cancer, skin cancer, including squamous cell carcinoma, esophagus cancer, kidney cancer, bladder cancer, gall cancer, cervical cancer, ovarian cancer, lung cancer, bronchial, small and non-small-cell lung cancer, gastric, and head and neck cancer.

Hematological tumors may be leukemia, such as Acute Myelogenous Leukemia (AML), Acute Lymphoblastic Leukemia (ALL), Acute Lymphocytic Leukemia, Acute Leukemia, Acute Promyelocytic Leukemia, Chronic Granulocytic Leukemia (CGL), Chronic Leukemia, Chronic Lymphocytic Leukemia (CLL), Chronic Myelogenous Leukemia (CML), Chronic Myelomonocytic Leukemia, Common-type Acute Lymphoblastic Leukemia, Eosinophilic Leukemia, Erythroleukemia, Extranodal Lymphoma, Follicular Lymphoma, Hairy Cell Leukemia, Monocytic Leukemia, Prolymphocytic Leukemia.

Hematological tumors may also be lymphoma, such as B Cell Lymphomas, Burkitt Lymphoma, Cutaneous T Cell Lymphoma, High-Grade Lymphoma, Hodgkin's Lymphoma, Non-Hodgkin's Lymphoma, Low-grade Lymphoma, Lymphoblastic Lymphoma, Mantle Cell Lymphoma, Marginal Zone Lymphoma, Mucosa-Associated Lymphoid Tissue (MALT) Lymphomas, T Cell Lymphomas, peripheral T cell lymphoma, multiple myeloma, Essential Thrombocythemia, Hairy Cell Lymphoma, Extramedullary myeloma, Granulocytic Sarcomae.

Hematological tumors may also be tumors of myeloid lineage, including acute and chronic myelogenous leukemias, myelodysplastic syndrome, and promyelocytic leukaemia.

Tumors may also be of mesenchymal origin, such as fibrosarcoma and rhabdomyosarcoma. Furthermore, tumors may be tumors of the central and peripheral nervous system, such as astrocytoma, neuroblastoma, glioma, and schwannomas; and tumors may be other tumors, such as melanoma, seminoma, teratocarcinoma, osteosarcoma, xenoderoma pigmentosum, keratoctanthoma, thyroid follicular cancer, and Kaposi's sarcoma.

Tumors that are resistant or refractory to treatment with other anti-cancer or anti-proliferative agents may also benefit from treatment with the methods and pharmaceutical compositions of the present invention.

Compounds disclosed herein may also be useful in the chemoprevention of cancer. Chemoprevention is defined as inhibiting the development of invasive cancer by either blocking the initiating mutagenic event or by blocking the progression of pre-malignant cells, such as by blocking growth of the tumor, that have already suffered an insult or inhibiting tumor relapse.

Compounds disclosed herein may also be useful in inhibiting tumor angiogenesis and metastasis.

The compounds of this invention may also be useful in combination (administered together or sequentially) with known anti-cancer treatments such as radiation therapy or with anti-cancer, anti-proliferative, cytostatic or cytotoxic agents. Other anti-cancer and anti-proliferative agents which may be used in combination with the compounds of the present invention include those described herein. In combination treatment, the compounds of the present invention may be further administered with any other anti-cancer and anti-proliferative agent disclosed herein.

If formulated as a fixed dose, such combination products employ the compounds of this invention within the dosage range described herein and the other pharmaceutically active agent or treatment within its approved dosage range. For example, the cdc2 inhibitor olomucine has been found to act synergistically with known cytotoxic agents in inducing apoptosis (J. Cell Sci., 108, 2897 (1995)). Compounds described herein may also be administered sequentially with known anti-cancer or anti-proliferative agents when a combination formulation is inappropriate. The invention is not limited in the sequence of administration; compounds described herein may be administered either prior to or after administration of the known anti-cancer or anti-proliferative agent. For example, the cytotoxic activity of the cyclin-dependent kinase inhibitor flavopiridol is affected by the sequence of administration with anticancer agents (Cancer Research, 57, 3375 (1997)).

### Further Aspects of the Invention

Another aspect the invention provides a pharmaceutical package, wherein said package includes a compound of any of the formulae of the present invention. In certain embodiments, the package comprises instructions which indicate that said composition may be used for the treatment of an individual in need thereof, including a human. In certain other embodiments, the pharmaceutical package includes one or more compounds of the present invention formulated together with another pharmaceutical ingredient such as an anti-cancer or anti-proliferative agent. In this case, the compound(s) of the present invention and the other pharmaceutical ingredient may be formulated separately and in individual dosage amounts.

Other pharmaceutical ingredients that may be formulated together or separately with the compounds of the present invention include but are not limited to other anti-cancer and anti-proliferative agents such as described above. In certain still further embodiments, the pharmaceutical package comprises instructions to treat a patient in need of such treatment. In yet another aspect the invention provides a pharmaceutical package for treating an individual suffering from a proliferative disorder or disease, such as a tumor or a cancer, wherein said package includes at least one compound of the present invention. In certain still further embodiments, the pharmaceutical package comprises instructions to treat the disorder.

As used herein the term "pharmaceutical package" or "pharmaceutical pack" refer to any packaging system for storing and dispensing individual doses of medication. Preferably the pharmaceutical package contains sufficient daily dosage units appropriate to the treatment period or in amounts which facilitate the patient's compliance with the regimen. In certain embodiments, the pharmaceutical pack comprises one or more vessels that include the active ingredient, e.g., a compound of the present invention. Such vessel can be a container such as a bottle, vial, syringe, or capsule, or may be a unit dosage form such as a pill. The active ingredient may be provided in the vessel in a pharmaceutically acceptable form or may be provided, for example, as a lyophilized powder. In further embodiments, the pharmaceutical pack may further include a solvent to prepare the active ingredient for administration. In certain embodiments, the active ingredient may be already provided in a delivery device, such as a syringe, or a suitable delivery device may be included in the pack. The pharmaceutical package may comprise pills, liquids, gels, tablets, dragees or the pharmaceutical preparation in any other suitable form. The package may contain any number of daily pharmaceutical dosage units. The package may be of any shape, and the unit dosage forms may be arranged in any pattern, such as circular, triangular, trapezoid, hexagonal or other patterns. One or more of the doses or subunits may be indicated, for example to aid the doctor, pharmacist or patient, by identifying such dose or subunits, such as by employing color-coding, labels, printing, embossing, scorings or patterns. The pharmaceutical package may also comprise instructions for the patient, the doctor, the pharmacist or any other related person.

Some embodiments comprise the administration of more than one active ingredient, including compounds as disclosed herein. Such administration may occur concurrently or sequentially. The active ingredients may be formulated together such that one administration delivers both components. Alternatively the active ingredients may be formulated separately. The pharmaceutical package may comprise the compound of the present invention and the other pharmaceutical ingredient in a single formulation, i.e., they are formulated together, or the compound of the present invention and the other pharmaceutical ingredient in individual formulations, i.e., they are formulated separately. Each formulation may comprise the compound of the present invention and the other pharmaceutical ingredient in individual dosage amounts (in approximately equal or unequal amounts). Administration of the compound of the present invention and the other pharmaceutical ingredient results in a concentration that results in a therapeutically effective amount of the combination.

As used herein, the term "instructions" means a product label and/or documents or other information describing relevant materials or methodologies pertaining to assembly, preparation or use of a kit or packaged pharmaceutical. These materials may include any combination of the following: background information, steps or procedures to follow, list of components, proposed dosages, warnings regarding possible side effects, instructions for administering the drug, technical support, and any other related documents. Instructions can be supplied in printed form, such as a package label or a package insert. Instructions for a packaged pharmaceutical or a pharmaceutical composition can be inserted in a delivery carton or finished package, e.g., as a package insert, and the text of such has been approved by a competent regulatory authority such as the Food and Drug Administration (FDA) of the United States. Alternatively or complementarily, instruction may also be stored in electronic form, e.g., on a computer-readable storage medium such as a computer-readable memory device, a centralized database, magnetic media such as hard disks, floppy disks, and magnetic tape; optical media such as compact discs, CD-ROMs and holographic devices; magneto-optical media such as floptical disks; and hardware devices that are specially configured to store and execute program code, such as application-specific integrated circuits (ASICs), programmable logic devices (PLDs) and ROM (read only memory) and RAM (random access memory) devices. Instructions may comprise a web address of an internet website from which more detailed instructions may be downloaded, or a recorded presentation. Instructions can contain one or multiple documents or future updates.

Another aspect of the invention provides the use of compounds of Formula (I), or any compound of the invention specifically disclosed herein, to inhibit one or more Polo-like kinases. In another embodiment of this aspect, any compound of Formula one is used for the preparation of a composition for the inhibition of one or more Polo-like kinases. In yet another embodiment, the invention provides methods to inhibit one or more Polo-like kinase. Each of such embodiments include those where the Polo-like kinase is Plk1.

Thus, in one aspect the invention relates to a pharmaceutical composition, including a compound of the present invention, and a pharmaceutically acceptable diluent, excipient or carrier.

In certain embodiments, such pharmaceutical composition comprises a therapeutically effective amount of said compound or prodrug.

In certain embodiments, such pharmaceutical composition is for the treatment of an individual in need thereof.

In certain embodiments of such pharmaceutical composition, said individual is a human.

In another aspect the invention relates to a pharmaceutical package, including a pharmaceutical composition of the present invention, and instructions which indicate that said pharmaceutical composition may be used for the treatment of an individual in need thereof.

In certain embodiments of such pharmaceutical package, said instructions indicate that said pharmaceutical composition may be used for the treatment of a human.

In certain embodiments of such pharmaceutical package, said instructions indicate that said pharmaceutical composition may be used for the treatment of an individual suffering from a proliferative disorder or disease.

In certain embodiments of such pharmaceutical package, said instructions indicate that said pharmaceutical composition may be used for the treatment of a human suffering from a proliferative disorder or disease.

In another aspect the invention relates to a method for treating a proliferative disorder or disease in an individual, comprising administering a therapeutically effective amount of a compound or a pharmaceutical composition of any the present invention.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for treating a proliferative disorder or disease in an individual, comprising exposing cells included in said disorder or disease to a compound of the present invention.

In certain embodiments of such method, said compound, or a prodrug thereof, is administered to said individual.

In certain embodiments of such method, said individual is a mammal selected from: domestic mammal, cat, dog, horse, sheep, cow, rodent, and human.

In certain embodiments of such method, said mammal is a human.

In another aspect the invention relates to a method for inhibiting cell proliferation, comprising contacting a cell with a compound of the present invention.

In another aspect the invention relates to a compound of any one of the present invention for the preparation of a medicament for the treatment of a proliferative disorder or disease.

In another aspect the invention relates to a pharmaceutical composition comprising a compound of of the present invention and a pharmaceutically acceptable carrier, diluent or excipient, for the treatment of a proliferative disorder or disease.

In certain embodiments of a pharmaceutical package, method, use or pharmaceutical composition of the present invention, the proliferative disorder or disease is a cancer.

The invention further relates to a method of synthesizing a compound according to the present invention, comprising the step of reacting a compound having a structure represented by formula (II) with 2,4-dichloro-5-nitro-pyrimidine 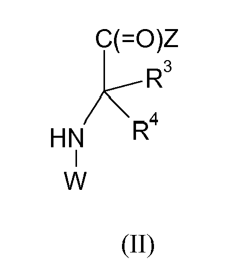 wherein W is selected from -O-R⁵ and -NH-P, wherein P is a protecting group, particularly: tert-butyloxycarbonyl (Boc), wherein Z is a leaving group, particularly -O-Me or O-Et, most particularly -O-Me, and wherein R³, R⁴ and R⁵ are as defined above.

In particular embodiments, W is -NH-P, particularly -NH-Boc.

Furthermore, the invention relates to a compound having a structure represented by formula (III) 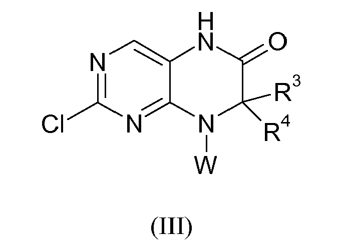 or any tautomeric or stereoisomeric form thereof, wherein R³, R⁴, and W are as defined above.

In particular embodiments, W is -NH-P, particularly -NH-Boc.

### Examples

A selection of compounds within the scope of the present invention is listed in Table 1, with representative data characterizing certain physicochemical properties being shown in Table 2. The compounds in Table 1 were synthesized according to Examples 1 to 4 below, and the surprising inhibitory activities in biochemical assays, and anti-proliferative activities in cellular assays of these compounds are shown in Table 3, as determined according to Examples 5 and 6.

### A: Synthesis of 6-oxo-6,7-dihydro-5H-pteridin-8-yl derivatives (examples 1-4)

Compounds of the invention may be prepared by the synthetic sequence shown in Scheme 1, as may be modified as described. A skilled artisan will appreciate that other routes of synthesis may be employed as well. In particular, other routes of synthesis may in fact be applied to certain aspects of the present invention. The skilled artisan is referred to general textbooks, such as March's Advanced Organic Chemistry (Michael B. Smith & Jerry March, Wiley-Interscience, 2000), The Practice of Medicinal Chemistry (Camile G. Wermuth, Academia Press, 2003) and Protective Groups in Organic Synthesis (Theosora W. Greene & Peter G.M. Wuts; John Wiley & Sons Inc, 1999).

Starting materials were commercially available or have been synthesized according to the following procedures:
Cyclobutane carboxaldehyde was prepared as described in WO2006/35195A1.
(Trans)-4-(4-(cyclopropylmethyl)piperazin-1-yl)cyclohexanamine, 3HCl was prepared according to WO2007/090844.
1'-Benzyl-1,4'-bipiperidin-4-amine, 3HCl: Tert-butyl 1'-benzyl-1,4'-bipiperidin-4-ylcarbamate was prepared according to WO2007/052938. Tert-butyl 1'-benzyl-1,4'-bipiperidin-4-ylcarbamate (320 mg, 0.857 mmol) was added to HCl (4M) in dioxane (4 ml) to give a suspension. The mixture was heated with microwave irradiation in a sealed vessel at 120°C for 2 min. The mixture was concentrated under reduced pressure. Yield: 320 mg.

### Physicochemical data of final compounds (see Table 2):

### Methods

The mass spectra (MS) data was generated on a LCMS system.

Method A:
HPLC component: Agilent 1200 system, Waters Xterra column 5x50 mm (3.5µm C18), 35°C, 0.1% formic acid aq. (SolvA) MeCN+0.1% formic acid v/v (SolvB), gradient 3%SolvB for 1.5 min, 3-70% SolvB 7 min; DAD 190-900 nm.
MS component: Waters ZQ.

Method B:
HPLC component: Agilent 1100 system, Zorbax Bonus column 4.6x75 mm (3.5µm C18), 35°C, 0.1% formic acid aq. (SolvA) MeCN+0.1% formic acid v/v (SolvB), gradient 5-95% SolvB 7 min, DAD 200-400nm.
MS component: Agilent 6120 Quadropole. 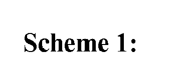 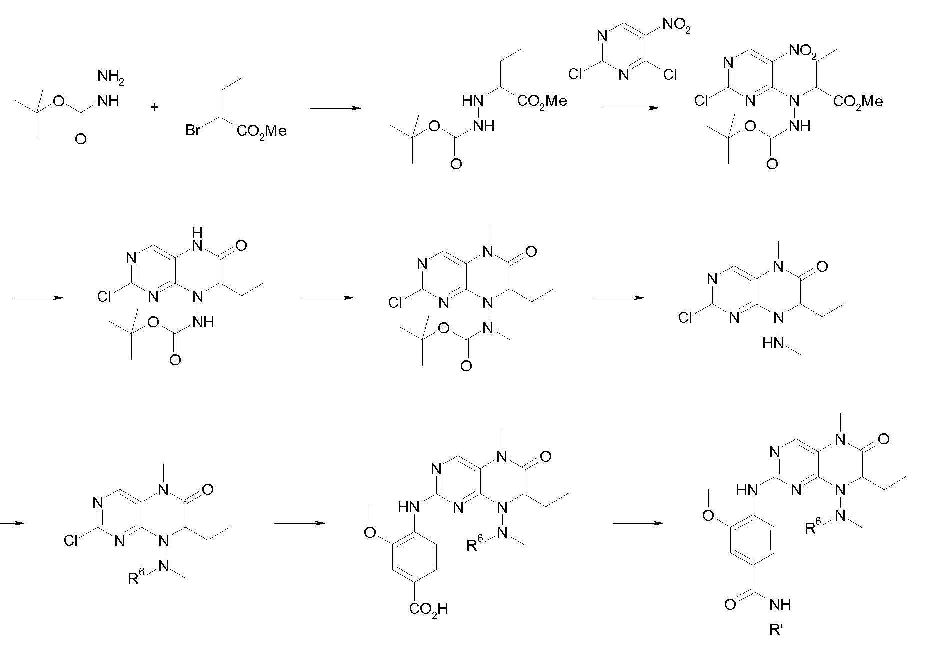
By using hydroxylamine derivatives instead of a protected hydrazine in the first synthesis step, the analogous compounds of the present invention with -X-R⁵ being -O-R⁵ can be obtained. Variability at R¹ can be obtained by using alternative arylamines or heteroarylamines in the nucleophilic replacement of chlorine in position 2 and/or by alternative modifications of the aryl or heteroaryl groups in the final step. Variability at R² and R⁵ (if -X-R⁵ is -N-(R⁵)-R⁶ can be obtained by using alternative alkylating reagents. According to Scheme 1, compounds being symmetric with regard to R² and R⁵ can be obtained. Compounds that are unsymmetric with regard to R² and R⁵ may be obtained by alkylating the nitrogen attached in position 8 with a first alkylating reagent prior to the cyclization reaction, followed after ring closure by functionalization (e.g. alkylation or formylation) of the ring nitrogen in position 6. Variability at R³ / R⁴can be obtained by using altenative α-bromo acetic acid ester derivatives in the first step. Variability at R⁶ (if present) can be obtained as shown above.

### Example 1

### 2-(N'-tert-Butoxycarbonyl-hydrazino)-butyric acid methyl ester

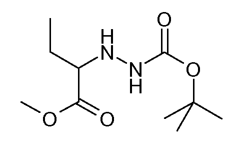

tert-Butyl carbazate (26.43 g; 0.2 mol) and DIPEA (28.43 g; 0.22 mol) were suspended in toluene (50 ml). Methyl-2-bromo-butyrate (36.21 g; 0.2 mol) was added dropwise at RT. After addition was complete, the mixture was heated under reflux for 7 h and left at RT for 3d. The mixture was diluted with Et₂O (250 ml) and stirred for 30 min. The precipitate was removed by filtration and washed with Et₂O (2 x 50 ml). The combined filtrate and washings were evaporated to dryness under reduced pressure and the residue was purified by flash chromatography (EtOAc/c-Hexane 1:3) to give 25.9 g (0.112 mol; 56%) of the title compound. Thin Layer Chromatography (TLC) (EtOAc/Hexanes 1:1): Rf = 0.50. MH⁺= 233.

### 2-[N'-tert-Butoxycarbonyl-N-(2-chloro-5-nitro-pyrimidin-4-yl)-hydrazino]-butyric acid methyl ester

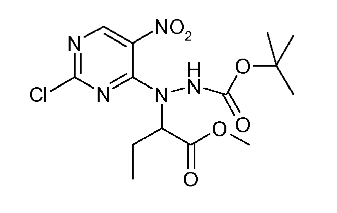

2-(N'-tert-Butoxycarbonyl-hydrazino)-butyric acid methyl ester (22.21g, 95.63 mmol) and K₂CO₃ (13.82 g; 100 mmol) was suspended in acetone (200 ml). Then 2,4-dichloro-5-nitropyrimidine (18.55 g; 95.63 mmol) was added dropwise at -35°C. After addition the reaction mixture was allowed to reach RT and stirring was continued for 20h. Then water (150 ml) was added and the mixture was extracted with EtOAc (3 x 150 ml). The combined organic extracts were washed with brine and dried over Na₂SO4. The solvent was removed under reduced pressure and the residue was purified by flash chromatography (EtOAc/c-Hexane 1:4) to give 35.9 g (92.1 mmol; 96%) of the title compound. TLC (EtOAc/Hexanes 1:4) Rf = 0.44. MH⁺ = 390; 392 (chlorine pattern)

### (2-Chloro-7-ethyl-6-oxo-6,7-dihydro-5H-pteridin-8-yl)-carbamic acid tert-butyl ester

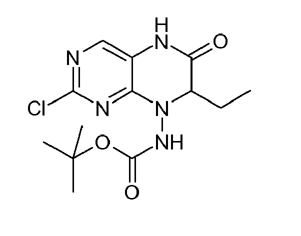

2-[N'-tert-Butoxycarbonyl-N-(2-chloro-5-nitro-pyrimidin-4-yl)-hydrazino]-butyric acid methyl ester (7218 mg; 18.5 mmol) was dissolved in EtOH (160 ml) and heated to 75°C. NH₄Cl (495 mg, 9.25 mmol) in water (100 ml) was added. Then Fe-powder (5171 mg, 92.6 mmol) was added portionwise. The mixture was stirred at 75°C for 20 min. The hot suspension was filtered through Celite. The red residue was washed with EtOH (4 x). The combined filtrate and washings were concentrated under reduced pressure to remove most of the EtOH. The aqueous suspension was extracted with EtOAc (1 x 320 ml), the organic extract was dried over Na₂SO₄ and filtered over Celite. The solvent was removed under reduced pressure to leave 30 g. Et₂O (50 ml) were added and the mixture was turned for 30 min. The precipitate was collected by filtration and washed with Et₂O to give 2730 mg of the title compound. The filtrate was evaporated to dryness and the residue was titurated with Et₂O (40 ml). The precipitate was collected by filtration and washed with Et₂O to give another 1g of the title compound. Yield: 3730 mg (11.4 mmol; 62%). TLC (EtOAc/Hexanes 1:1): Rf = 0.42. MH⁺ = 328; 330

### (2-Chloro-7-ethyl-5-methyl-6-oxo-6,7-dihydro-5H-pteridin-8-yl)-methyl-carbamic acid tert-butyl ester

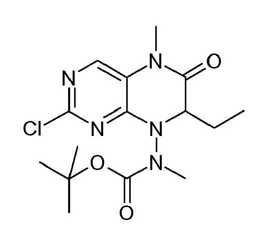

(2-Chloro-7-ethyl-6-oxo-6,7-dihydro-5H-pteridin-8-yl)-carbamic acid tert-butyl ester (2106 mg; 6.42 mmol) and K₂CO₃ (2391 mg ; 17.3 mmol) were suspended in DMF (12 ml) at RT. Methyl iodide ( 2100 mg; 14.8 mmol) were added dropwise. The mixture was stirred at RT for 5h. Residual methyl iodide was removed under reduced pressure and the mixture was poured into water (120 ml). The mixture was stirred and then centrifuged and the water was discarded. The residual oil was dissolved in dichloromethane (150 ml). The solution was washed with brine (100 ml) and dried over Na₂SO₄. After filtration the solvent was removed under reduced pressure to give 2158 mg (6.06 mmol, 94 %) of the title compound. TLC (EtOAc/Hexanes 1:1) Rf = 0.23, 0.39 (two spots). MH⁺ = 356; 358

### 2-Chloro-7-ethyl-5-methyl-8-methylamino-7,8-dihydro-5H-pteridin-6-one

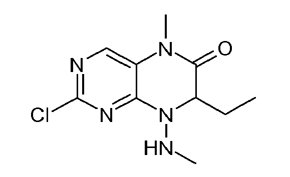

HCl was bubbled through i-PrOH (150 ml) for 45 min under ice-cooling. Then (2-chloro-7-ethyl-5-methyl-6-oxo-6,7-dihydro-5H-pteridin-8-yl)-methyl-carbamic acid tert-butyl ester (10.28 g; 28.9 mmol) was added and the mixture was stirred at 66°C for 1h. After cooling to RT the resulting precipitate was collected by filtration, washed with Et2O and dried in vacuo to give 8.0 g (24.3 mmol; 84%) white solid. To isolate the free base, the solid was suspended in dichloromethane (500 ml) and washed with sat. NaHCO₃-solution (150 ml). The organic layer was dried over Na₂SO₄, filtered and the solvent was removed under reduced pressure to give 6.22 g (24.3 mmol; 84%) of the title compound. TLC (EtOAc/Hexanes 1:1): Rf = 0.22. MH⁺ = 256; 258.

### 2-Chloro-8-dimethylamino-7-ethyl-5-methyl-7,8-dihydro-5H-pteridin-6-one

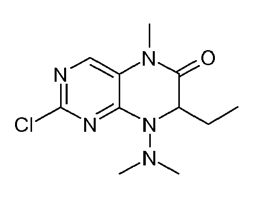

2-Chloro-7-ethyl-5-methyl-8-methylamino-7,8-dihydro-5H-pteridin-6-one (1195 mg; 4.67 mmol), formaldehyde (1.9 ml 37% aq. solution; 11.7 mmol) and HOAc (392 mg; 6.5 mmol) were dissolved in acetonitrile (20 ml) and stirred for 45 min. Sodium cyanoborohydride (890 mg; 14.2 mmol) in acetonitrile (20 ml) was added dropwise under cooling. The mixture was stirred for 15h at RT. Then formaldehyde (1.0 ml 37% aq. solution; 6.2 mmol), and HOAc (200 mg; 3.3 mmol) were added and the mixture was stirred for 1h. Sodium cyanoborohydride (480 mg; 7.6 mmol) was added and stirring was continued for 1h. The mixture was diluted with EtOAc (100 ml) and washed with NaHCO₃-solution (50 ml). The aqueous layer was back-extracted with EtOAc (1 x 30 ml), the combined organic layers were washed with brine (2 x 20 ml), dried over Na₂SO₄, filtered and evaporated to dryness. The residue was purified by flash chromatography to give 605 mg (2.24 mmol; 48%) of the title compound. TLC (2 x EtOAc/Hexanes 1:1): Rf = 0.5. MH⁺ = 270; 272.

### 4-(8-Dimethylamino-7-ethyl-5-methyl-6-oxo-5,6,7,8-tetrahydro-pteridin-2-ylamino)-3-methoxy-benzoic acid

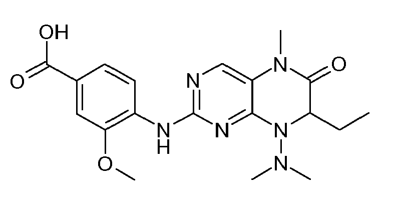

To a white suspension of 2-chloro-8-dimethylamino-7-ethyl-5-methyl-7,8-dihydro-5H-pteridin-6-one (1460 mg, 5.41 mmol) in acetonitrile (25 ml) was added 4-amino-3-methoxybenzoic acid (905 mg, 5.41 mmol) at RT followed by 2M aq. HCl (5.4 ml, 10.8 mmol). The mixture was heated under reflux (oil bath temp 90°C) for 35 h. The mixture was cooled to RT. The precipitate was collected by filtration, washed with MeOH (5 ml) and Et2O ( 5 ml) to give 885 mg of the title compound. The filtrate was heated under reflux for another 3 d and treated as above to give another 335 mg of the title compound. Total yield: 1220 mg (3.05 mmol, 56%). TLC (EtOH : Toluene : NH₃conc = 4:5:1): Rf = 0.32. MH⁺ = 401.

### 4-(8-Dimethylamino-7-ethyl-5-methyl-6-oxo-5,6,7,8-tetrahydro-pteridin-2-ylamino)-3-methoxy-N-(1-methyl-piperidin-4-yl)-benzamide (compound 1; racemate)

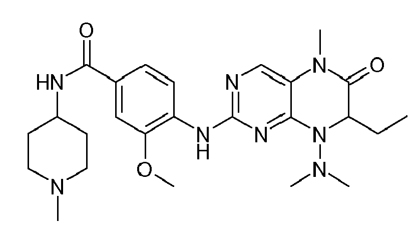

To a white suspension of 4-(8-dimethylamino-7-ethyl-5-methyl-6-oxo-5,6,7,8-tetrahydropteridin-2-ylamino)-3-methoxy-benzoic acid (86 mg, 0.215 mmol) in dichloromethane (15 ml) was added DIPEA (0.030 mg, 0.235 mmol) at RT followed by TBTU (72 mg, 0.225 mmol). The mixture was stirred at RT for 30 min. Then 1-methylpiperidin-4-amine (26 mg, 0.228 mmol) in dichloromethane (2 ml) was added dropwise and the mixture was stirred at RT for 3d. The mixture was washed with water (1 x 1 ml), sat. NaHCO₃-solution (6 x 10 ml) and brine (1 x 10 ml). The organic solution was dried over Na₂SO₄, filtered and the solvent was removed under reduced pressure. The residue was purified by flash chromatography (dichloromethane / MeOH, 6:1 -> 5:1) to give 80 mg (0.161 mmol; 75%) of the title compound. MH⁺ = 497.

### Example 2

### 4-(8-Dimethylamino-7-ethyl-5-methyl-6-oxo-5,6,7,8-tetrahydro-pteridin-2-ylamino)-N-(1-ethyl-piperidin-4-ylmethyl)-3-methoxy-benzamide

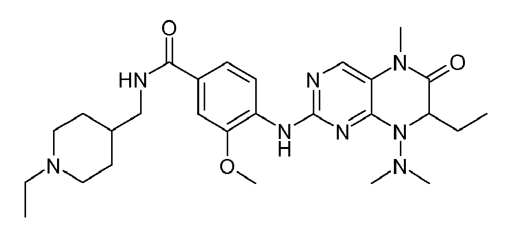

To a suspension of 4-(8-(dimethylamino)-7-ethyl-5-methyl-6-oxo-5,6,7,8-tetrahydropteridin-2-ylamino)-3-methoxybenzoic acid (50.0 mg, 0.125 mmol) in abs. CH₂Cl₂ (5 ml, 40 ml/mmol) was added DIPEA (40.3 mg, 0.312 mmol) at RT to give a solution. HATU (57.0 mg, 0.150 mmol) was then added and the reaction was stirred under N₂ at RT for 1h. C-(1-Ethyl-piperidin-4-yl)-methylamine (2.60 mg, 0.162 mmol) was added and the reaction was stirred under N₂ at RT overnight.

The reaction mixture was concentrated, the residue was dissolved in MeOH (1 ml) and purified by preparative HPLC to give 29 mg (0.055 mmol; 44%) of the title compound. MH⁺= 525.

### Example 3

### 2-Chloro-8-((cyclopentylmethyl)(methyl)amino)-7-ethyl-5-methyl-7,8-dihydropteridin-6(5H)-one

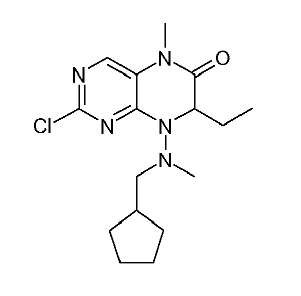

To a colorless suspension of 2-chloro-7-ethyl-5-methyl-8-(methylamino)-7,8-dihydropteridin-6(5H)-one (420 mg, 1.643 mmol) in ethanol (8 ml) was added cyclopentanecarbaldehyde (806 mg, 8.21 mmol) at RT followed by HOAc (0.940 ml, 16.43 mmol). The mixture was stirred at RT for 1 h. Then sodium cyanoborohydride (516 mg, 8.21 mmol) was added portionwise over 2h at 0°C under stirring. The ice bath was removed and stirring was continued for 1h at RT, when HPLC-MS showed about 60% conversion and the formation of a side product (5%). The reaction was quenched by addition of water (20 ml) and sat. aq. NaHCO₃ solution (30 ml). The mixture was extracted with EtOAc (2 x 50 ml). The org. extracts were combined and dried over Na₂SO₄, filtered and concentrated under reduced pressure. The residue (1.12g oil) was purified *via* flash chromatography (10 -> 50% EtOAc in c-Hexane over 25 min).

The product containing fractions were pooled, evaporated to dryness and the residue was dried for 6h at 0.14 mbar to give the title compound (316 mg; 0.935 mmol; 57 %). TLC (EtOAc/c-Hexane 1:1): Rf = 0.5. MH⁺ = 338; 340

### 4-(8-((Cyclopentylmethyl)(methyl)amino)-7-ethyl-5-methyl-6-oxo-5,6,7,8-tetrahydropteridin-2-ylamino)-3-methoxybenzoic acid

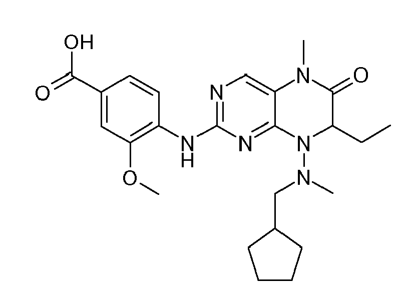

To a white suspension of 2-chloro-8-((cyclopentylmethyl)(methyl)amino)-7-ethyl-5-methyl-7,8-dihydropteridin-6(5H)-one (312 mg, 0.923 mmol) in acetonitrile (4.5 ml) was added 4-amino-3-methoxybenzoic acid (154 mg, 0.923 mmol) at RT followed by 2 M aq. HCl (0.923 ml, 1.847 mmol). The mixture was stirred at reflux (oil bath temp 90°C) for 20 h, when TLC (EtOAc/c-hexane 1:1) showed starting material left and the formation of one major product. The mixture was evaporated to dryness, the residue was suspended in iPrOH (3 ml), the mixture was heated to reflux and sonicated. The precipitate was collected by filtration, washed with iPrOH (2 x 1ml) and dried in vacuo to give 208 mg (0.443 mmol; 48%) of the title compound. MH⁺ = 469

### 4-(8-((Cyclopentylmethyl)(methyl)amino)-7-ethyl-5-methyl-6-oxo-5,6,7,8-tetrahydropteridin-2-ylamino)-3-methoxy-N-(1-methylpiperidin-4-yl)benzamide

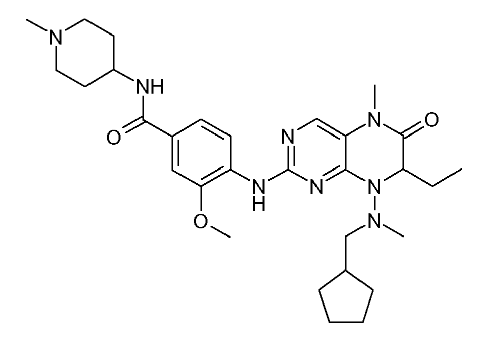

To a white suspension of 4-(8-((cyclopentylmethyl)(methyl)amino)-7-ethyl-5-methyl-6-oxo-5,6,7,8-tetrahydropteridin-2-ylamino)-3-methoxybenzoic acid (40 mg, 0.085 mmol) in DCM (3 ml) was added HATU (42.2 mg, 0.111 mmol) at RT followed by DIPEA (0.030 ml, 0.171 mmol). To the resulting solution 1-methylpiperidin-4-amine (13 mg, 0.111 mmol) was added and the mixture was stirred at RT for 20 h. HPLC-MS showed no starting acid left and the formation of one product with desired mass [m/z = 283 (MH₂ ²⁺)]. The solvent was removed under reduced pressure, the residue was dissolved in MeOH (1.0 ml) and purified by prep HPLC-MS.

The fractions were pooled, MeCN was removed under reduced pressure and the product was freeze dried to give 34.4 mg (0.061mmol; 71 %) of the title compound. MH⁺ = 565

The compounds in Table 1 were prepared in an analogous manner using the same reaction sequence and corresponding aldehydes and amines, respectively.

### Example 4

### Enantiomeric separation of Compound 1 by preparative HPLC

About 115 mg of racemic compound 1 were separated into the pure enantiomers by preparative HPLC.

Preparative method:
Column: CHIRALPAK® IC 5 µm - 250 x 50 mm
Mobile phase: n-heptane / ethanol / diethylamine 70/30/0.1 (v/v/v)
Flow rate: 100 ml/min
Detection: UV 340 nm Temperature: 25°C

Analytical method:
Column: CHIRALPAK® IC 5 µm - 250 x 4.6 mm
Mobile phase: n-heptane / ethanol / diethylamine 70/30/0.1 (v/v/v)
Flow rate: 1 ml/min
Detection: UV 340 nm
Temperature: 30°C

### Results:

The two enantiomers were obtained in amounts of 50 mg each. The first eluting enantiomer (compound 36) had a retention time of 19.6 min, the second (compound 37) of 25.5 min. The chemical purity (measured as area% at 340 nm) and the enantiomeric excess were greater than 99% in all cases.

### B: Biological Activity Assays (examples 5 to 7)

The biological activity and utility of the compounds of the invention are demonstrated by one or more assays including those described in more detail below.

### Example 5: Biochemical assays for inhibition of kinase activity

### a) Plk1 kinase assay

| Kinase: PLK1 fl-His (Invitrogen; Cat.No. PV4038) | |
|---|---|
| IMAP Assay: | |
| Reaction Volume: | 8 µl |
| Reaction Time: | 60 min |
| Reaction Temperature: | room temperature |
| IMAP Incubation Time: | 60 min |
| Assay Plate: | 384 well U bottom, PP, black, low volume (Coming, 3676) |
| Compound Plate: | 384 well U bottom, PS (Falcon, 3995) |
| IMAP Binding Buffer A: | Molecular Devices, R7282 |
| IMAP Binding Buffer B: | Molecular Devices, R7283 |
| IMAP Binding Reagent: | Molecular Devices, R7207 |

| Controls: | |
|---|---|
| Negative Control (C-): | no kinase, no inhibitor |
| Positive Control (C+): | no inhibitor |
| Reaction buffer: | 20 mM Tris, pH 7.0 |
| | 1 mM DTT |
| | 2 mM MgCl₂ |
| | 2 mM MnCl₂ |
| | 0.01 % Tween20 |

| Final Assay Concentrations: | |
|---|---|
| Kinase: | Kinase conc. yielding 50% substrate turnover as determined in titration experiment PLK1 fl-His (Invitrogen; PV4038) |
| ATP: | 0.63 µM |
| Substrate: | 5FI-RGSFNDTLDFD-NH2 400 nM (jpt Peptide Technologies GmbH, Berlin, Germany) |

| IMAP Binding Solution: | 40 % IMAP Binding Buffer A |
|---|---|
| | 60 % IMAP Binding Buffer B |
| | IMAP Binding Reagent 1:1200 |

### Pipetting Sequence:

1) Add 6 µl 1.33-fold concentrated substrate + 1.33-fold concentrated ATP in 1-fold concentrated reaction buffer to each well of assay plate
2) Add 10.8 nl 740-fold concentrated inhibitor in 100% DMSO to each well except to C-and C+ wells using pintool (CyBio, Jena, Germany) (starting point: final inhibitor concentration 10 µM; IC₅₀ determination based on dilution series)
3) Add 10.8 nl 100% DMSO to C- and C+ wells using pintool
4) Add 2 µl reaction buffer to C- wells
5) Add 2 µl 4-fold concentrated kinase in reaction buffer to each well except C- wells
6) Incubate according to reaction time at room temperature
7) Add 15 µl IMAP binding solution to each well
8) Incubate according to IMAP incubation time at room temperature
9) Measure fluorescence polarization using integration time of 0.1 s (Analyst GT, Molecular Devices)

### b) Select Screen™ Kinase Assay

Selected compound of the present invention were tested in the Select Screen™ Kinase Profiling Service (Invitrogen Corporation, Madison, WI 53719, USA) against Plk1, under conditions of non-disclosure, non-dissemination and non-use.

In brief, compounds 1 and 13 (see Table 1) were tested at Invitrogen, and IC₅₀ values against Plk1 activity of about 20 nM were obtained in each case.

### c) Selectivity Panel:

Selected compound of the present invention were tested in a standard selectivity panel including Plk1 and 23 other kinases (Proteros biostructures GmbH, Martinsried/Munich, Germany), under conditions of non-disclosure, non-dissemination and non-use.

In brief, compounds 1, 13, 14, and 25 (see Table 1) were tested at 1 mM in triplicates against Plk1 as well as against Abl, Akt1, Aur A, c-Kit, c-Raf, Cdk1/CycB, Chk1, FGFR3, Flt3, Fyn, IGF1-R, INSR, Lyn, MAPK-ERK1, p38a, PAK4, PKCalpha, Ret, ROCK2, RSK 1, c-Src, TIE2, and VEGF-R2. All four test compounds inhibited Plk1 from about 50% to about 65%, all other kinases were inhibited by less than 10% in every case.

### Example 6: In vitro kinase activity of compounds of the present invention in cancer cell line

We observed the surprising finding that compounds of the invention were able to penetrate cell membranes and retained their activity as Plk1 inhibitors against native Plk1 expressed within living cancer cells.

Cellular Plk1 kinase activity was measured by quantification of nucleophosmin (B23) Ser4 phosphorylation in a cancer cell line. Nucleophosmin (B23) Ser4 is phosphorylated by Plk1, and this phosphorylation can be specifically detected by cross-reactivity of a phospho-Mek1/2 antibody that is able to recognizes a mitotic-specific phosphorylated form of nucleophosmin (B23) in a nocodazole dependent way (Zhang, H., Shi, X., Paddon, H., Hampong, M., Dai, W., and Pelech, S. (2004) B23/Nuleophosmin Serin 4 Phosphorylation Mediates Mitotic Functions of Polo-like Kinase 1. J. Biol. Chem., 279(34), 35726-35734).

Briefly, HCT116 cells (ATCC, Order No. CCL-247) were plated in 96 well dishes 24 hours prior to nocodazole treatment at 25 000 cells per well. Cells were then treated with 100 ng/ml nocodazole (Sigma-Aldrich, # M1404). 20 hours after nocodazole treatment, cells were exposed to the test compounds at various concentrations appropriate to determine an IC₅₀. 4 hours after compound addition, medium was carefully removed and 120 µl of Complete Tris Lysis Buffer (R60TX; MSD, Gaithersburg MD) was added to each well. Samples were then stored at -20°C.

The level of nucleophosmin (B23) Ser4 phosphorylation in these cell lysis samples was estimated by electrochemiluminescence detection using the Meso Scale Discovery ("MSD") Technology Platform (MSD, Gaithersburg MD). The protocol used was as recommended by the manufacturer with the use of a phospho-Mek1/2 antibody (Cell Signalling, #9121) as primary antibody, and is as follows.

The cell lysates were thawed at room temperature and 40 µl were incubated on 96-well MULTI-ARRAY High Bind plates (L11XB; MSD) for 1.5 - 2 hours at room temperature. The supernatants were then removed and 150 µl of a 5% (w/v) solution of Blocker A (R93AA; MSD) in Tris Wash Buffer (R61TX; MSD) were added to each well. The plates were then incubated for 1 hour at room temperature before removal of the Blocker A solution and washed 4 times with 200 µl Tris Wash Buffer. 25 µl primary antibody (anti-phospho-MEK1/2, Cell Signalling, #9121, rabbit polyclonal), diluted 1:250 in a 1% (w/v) solution of Blocker A in Tris Wash Buffer, were added to each well. The plates were then incubated for 1 hour at room temperature before removal of the antibody solution and washed 4 times with 200 µl Tris Wash Buffer. Then, 25 µl of labelled secondary antibody (SULFO-TAG Goat Anti-Rabbit IgGAntibody (R32AB; MSD), diluted 1:500 in a 1% (w/v) solution of Blocker A in Tris Wash Buffer) were added to each well. The plates were then incubated for 1 hour at room temperature before removal of the antibody solution and washed 4 times with 200 µl Tris Wash Buffer. Then, 150mµl of 1x Read Buffer T (R92TC; MSD) were carefully added to each well and the plates were quantitatively detected for each well with a SECTOR™ Imager 6000 (MSD).

Inhibitory activity of the compounds (IC) was calculated as % inhibition of nucleophosmin (B23) Ser4 phosphorylation compared to cells not treated with nocodazole. Table 3 represents the IC₅₀ values for inhibition of phosphorylation of nucleophosmin (B23) Ser4 for certain compounds of the invention showing that the compounds demonstrated a clear ability to inhibit such phosphorylation, and hence act as inhibitors of native Plk1 in a cancer cell line.

### Example 7: In vitro anti-proliferative activity of compounds of the present invention against cancer cell lines

We observed the surprising finding that compounds of the invention were useful in inhibiting proliferation of tumor cell lines (see Table 3).

**Table 7.1: Cell Lines used for demonstration of anti-proliferative activity**

| ATCC Name | ATCC Order No. | Doubling Time Td[hours] | Cells/ well |
|---|---|---|---|
| HCT 116 | CCL-247 | 15 | 3500 |
| HT-29 | HTB-38 | 20 | 9000 |
| IMR-90 | CCL-186 | 53 | 8000 |

Cells were exposed to the test compounds at various concentrations appropriate to determine an IC₅₀ for 72 hours. Cell proliferation was measured using the SRB assay according to Shekan et al (J Natl Cancer Inst (1990) 82, 1107-112) in order to estimate the IC₅₀ values shown in Table 3. Briefly, cells were plated in 96 well dishes 24 hours prior to compound addition at the densities specified in Table 5.1. The assay was terminated with the addition of cold TCA to a final concentration of 10% after growth medium has been removed and the plates were incubated over night at 4°C. The plates were then washed 5 times with water and 100µl of a Sulforhodamine B solution (4%) was added to each well. The plates were then incubated for 10 minutes at room temperature before removal of unbound dye by washing with 1% acetic acid. The bound dye was solubilised with 10 mM Trizma base and the absorbance read at OD520.

Inhibitory activity of the compounds (IC) was calculated as % inhibition of cell proliferation compared to cells treated with the solvent (DMSO). Table 3 represents the IC₅₀ values for inhibition of cell proliferation for certain compounds of the invention showing that the compounds demonstrated a clear and pronounced anti-proliferative activity towards rapidly cycling cancer cell lines (HCT 116 and HT-29) compared to the slow cycling cell line (IMR90).

### Example 8: In vitro anti-proliferative activity against cell-lines representing a variety of cancer types

We observed that compounds of the invention were surprisingly able to inhibit the proliferation of a variety of tumour cell-lines representing different types of cancers.

IC₅₀ estimates for BI 2536 and for compounds 1 and 13 (see Table 1) of the invention for inhibition of proliferation of the cell-lines shown in Table 8.1 (representing the various cancer types listed therein) were determined using the method of the previous example, with the appropriate number of cells per well.

**Table 8.1: 18-Cell Line Panel**

| ATCC Name | Cancer origin | ATCC Order No. | Cells/well |
|---|---|---|---|
| A-431 | skin | CRL-1555 | 9000 |
| A549 | lung | CCL-185 | 2500 |
| ACHN | kidney | CRL-1611 | 6000 |
| DU 145 | prostate | HTB-81 | 6000 |
| MDA-MB-468 | breast | HTB-132 | 8000 |
| NCI-H460 | NSCLC | HTB-177 | 3000 |
| OVCAR-3 | ovary | HTB-161 | 8000 |
| PC-3 | prostate | CRL-1435 | 4000 |
| SW620 | colon | CCL-227 | 9000 |
| A-375 | melanoma | CRL-1619 | 3500 |
| HT-29 | colon | HTB-38 | 9000 |
| HCT 116 | colon | CCL-247 | 4000 |
| HeLa | cervix | CCL-2 | 9000 |
| IMR-90 | lung | CCL-186 | 8000 |
| MDA-MB-231 | breast | HTB-26 | 9000 |
| SK-MEL-28 | melanoma | HTB-72 | 9000 |
| SK-MEL-5 | melanoma | HTB-70 | 3500 |
| COLO 205 | colon | CCL-222 | 9000 |

Table 8.2 shows that compounds of the invention are able to inhibit the proliferation of a broad range of tumour cell-lines that represent different types of cancers. Indeed, despite their structural difference, representative compounds of the invention are surprisingly able to inhibit the proliferation of as general a broad class of cell-lines as the compound BI 2536, the only Plk inhibitor to have undergone clinical testing to date.

**Table 8.2: Results from 18-Cell Line Panel**

| ATCC Name | IC₅₀[µM] | | |
|---|---|---|---|
| | Compound 1 | Compound 13 | BI 2536 |
| A-431 | <1 | <1 | <1 |
| A549 | <1 | <1 | <1 |
| ACHN | >1 | <1 | <1 |
| DU145 | <1 | <1 | <1 |
| MDA-MB-468 | < 1 | > 1 | *plateau at* ∼*50%* |
| NCI-H460 | <1 | <1 | <1 |
| OVCAR-3 | <1 | <1 | <1 |
| PC-3 | <1 | <1 | <1 |
| SW620 | <1 | <1 | <1 |
| A-375 | <1 | <1 | <1 |
| HT-29 | <1 | <1 | <1 |
| HCT116 | <1 | <1 | <1 |
| HeLa | <1 | <1 | <1 |
| IMR-90 | >1 | >1 | >1 |
| MDA-MB-231 | >1 | <1 | <1 |
| SK-MEL-28 | >1 | >1 | <1 |
| SK-MEL-5 | <1 | <1 | <1 |
| COLO 205 | <1 | <1 | <1 |

### Example 9: Activity of compounds in xenograft tumor models

With this assay we demonstrate the activity of compounds of the present invention against tumor cells using an in-vivo xenograft model.

### METHODS:

### Compound preparation

Compounds of the present invention (see Table 1) are prepared for i.p. administration in a biocompatible vehicle (e.g. in 10% DMA/50% PEG300/water; or in 5% Cremophor EL/5% ethanol/90% saline). All preparations are made freshly and injection volumes are adjusted to body weight (resulting in compound dosages of, for example, between 40 and 80mg/kg mouse).

### Mice/Husbandry.

Mice are obtained from Charles River Laboratory (CRL), housed in static microisolators, and provided *ad libitum* with water and an irradiated standard rodent diet (Purina Pico-Lab Rodent Diet 20).

### Standard Protocol.

### Experiments in Athymic mice.

Athymic nu/nu female mice (6-8 weeks old) from CRL are allowed to acclimate for at least 4 days. Human HT-29 cells (ATCC) colon carcinoma cell line are cultured in McCoy's medium (ATCC) supplied with 10% FCS and 1% Pen/Strep. The 2nd passage of cells with approximately 80% confluence is used in the study. Briefly, on Day 0, mice are inoculated with 0.1 ml (total 5x10⁶ cells) of HT-29 cell suspension (50x10⁶ cells/ml in McCoy's medium without supplements mixed 1:1 with Matrigel) by a subcutaneous injection into the lower right flank under light anesthesia. When the average tumor weights reaches above 100mg, animals with an appropriate tumor size are selected and are randomly divided into treatment groups (10 animals each).

Tumor growth and body weight are monitored and recorded twice a week. Tumors are measured by determining the length and width of the tumor with a digital caliper. Tumor weight is estimated using the following formula: Tumor Weight (mg) = (w² x 1) / 2 where w = width and I = length in mm of the tumor.

Tumor Growth Inhibition (TGI) % is calculated as follows: %TGI = 100(1-T/C) where T is the mean tumor size of a compound treated group on a given day, and C is the mean tumor size of the vehicle control group on the same day.

Toxic deaths are defined as deaths caused by compound treatment and not by advanced disease state. A death is considered toxic if the animal dies within 1 week after the final compound treatment and the tumor size has not reached 1000 mg non-tumor related deaths after this point are recorded, but not considered toxic deaths.

One group is treated with vehicle only (e.g. 10%DMA/50% PEG300/40% ddwater, *po*), and three groups of mice per compound and treatment schedule are treated with the respective compound in increasing amounts (e.g. with 40, 60 and 80 mg/kg doses dosed for five or ten days).

Mice are sacrificed when their tumors reach the 1000 mm³ endpoint volume. Treatment efficacy is determined as Log Cell Kill (LCK). LCK is a calculation that determines the percentage of tumor cells that are presumably killed after the initiation of treatment and can be used as a quantitative measure of efficacy: LCK = (T-C) / (3.32)(Td) where T = is the mean time required for the treatment group of mice to reach 1000 mg in size, C = the mean time for the control group tumors to reach 1000 mg in size, Td = is the Tumor Doubling time estimated from the linear regression analysis from a semi-log growth plot of the control group tumors during exponential growth and 3.32 = the number of doublings required for a population to increase 1-log10 unit. Each LCK unit represents 1-log10 unit of cell killing (e.g., 1 LCK = 90% kill, 2 LCK = 99% kill, etc.).

### C: Selection and development of drug candidates

### Example 10

In order to select the most appropriate compound to enter further experiments and to assess its suitability for use in a therapeutic composition for the treatment of disorders and diseases, such as cancers, additional data are collected. Such data can include the *in vitro* inhibition of the target molecule, such as a kinase, as measured by IC₅₀, or inhibition of proliferation across a panel of tumor cell lines, and tumor growth inhibition or reduction data and survival data from *in vivo* animal models. Furthermore, such experiments may also include the elucidation and/or determination of the mechanism of action of the subject compound, the target or target profile of the subject compound, and other characteristics of the subject compound, such as the binding affinity of the compound to the target(s) or the binding site of the compound on the target(s) and pharmacokinetic properties. Such experiments may also include molecular modelling of the drug-target interaction and the identification of metabolites formed after administration.

The compound that shows the most appropriate results for IC₅₀ for target inhibition, inhibition of cell proliferation, spectrum across various tumor cell lines, inhibition of tumour growth or tumour reduction data and/or animal-survival data, and/or other features, including ADME, pharmacokinetic and pharmacodynamic properties, may be chosen to enter further experiments. Such experiments may include, for example, therapeutic profiling and toxicology in animals, phase I clinical trials in humans and other clinical trails.

One skilled in the art readily appreciates that the present invention is well adapted to carry out the objects and obtain the ends and advantages mentioned, as well as those inherent therein. The methods and reagents described herein are representative of preferred embodiments, are exemplary, and are not intended as limitations on the scope of the invention. Modifications therein and other uses will occur to those skilled in the art. These modifications are encompassed within the spirit of the invention and are defined by the scope of the claims.

Those skilled in the art will recognize, or be able to ascertain using no more than routine experimentation, many equivalents to the specific embodiments of the invention described herein. Such equivalents are intended to be encompassed by the following claims. Those skilled in the art will also recognize that all combinations of embodiments, combination of aspects or features of the claims described herein are within the scope of the invention.

**Table 1: Exemplary compounds of the present invention**

| No. | | |
|---|---|---|
| 1 | 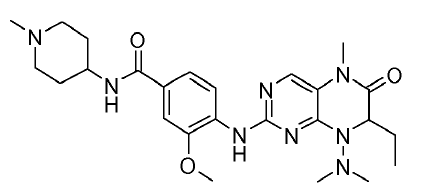 | 4-(8-Dimethylamino-7-ethyl-5-methyl-6-oxo-5,6,7,8-tetrahydro-pteridin-2-ylamino)-3-methoxy-N-(1-methyl-piperidin-4-yl)-benzamide (7-(R,S)-racemate) |
| 2 | 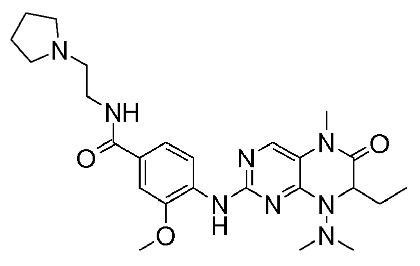 | 4-(8-Dimethylamino-7-ethyl-5-methyl-6-oxo-5,6,7,8-tetrahydro-pteridin-2-ylamino)-3-methoxy-N-(2-pyrrolidin-1-yl-ethyl)-benzamide |
| 3 | 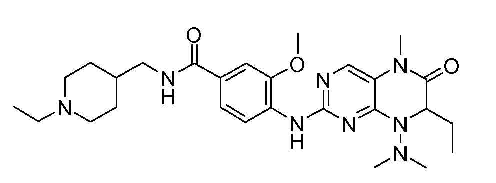 | 4-(8-Dimethylamino-7-ethyl-5-methyl-6-oxo-5,6,7,8-tetrahydro-pteridin-2-ylamino)-N-(1-ethyl-piperidin-4-ylmethyl)-3-methoxy-benzamide |
| 4 | 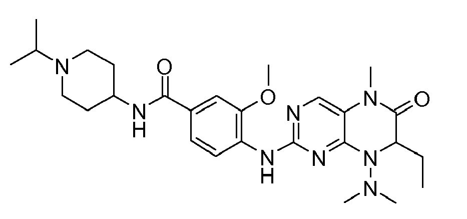 | 4-(8-Dimethylamino-7-ethyl-5-methyl-6-oxo-5,6,7,8-tetrahydro-pteridin-2-ylamino)-N-(1-isopropyl-piperidin-4-yl)-3-methoxy-benzamide |
| 5 | 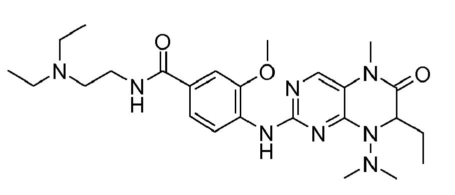 | N-(2-Diethylamino-ethyl)-4-(8-dimethylamino-7-ethyl-5-methyl-6-oxo-5,6,7,8-tetrahydro-pteridin-2-ylamino)-3-methoxy-benzamide |
| 6 | 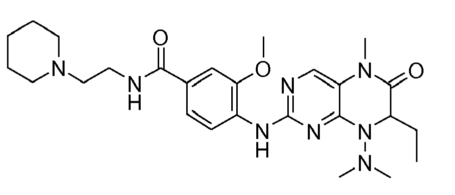 | 4-(8-Dimethylamino-7-ethyl-5-methyl-6-oxo-5,6,7,8-tetrahydro-pteridin-2-ylamino)-3-methoxy-N-(2-piperidin-1-yl-ethyl)-benzamide |
| 7 | 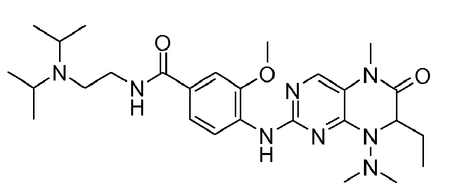 | N-(2-Diisopropylamino-ethyl)-4-(8-dimethylamino-7-ethyl-5-methyl-6-oxo-5,6,7,8-tetrahydro-pteridin-2-ylamino)-3-methoxy-benzamide |
| 8 | 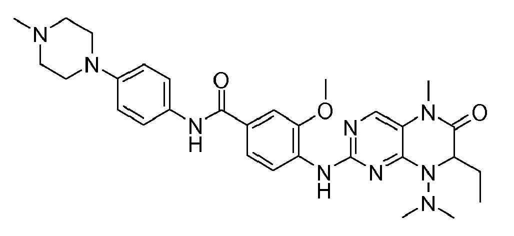 | 4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-phenyl]-benzamide |
| 9 | 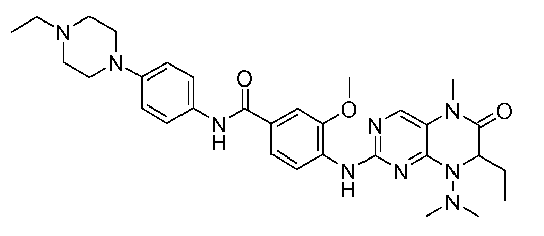 | 4-(8-Dimethylamino-7-ethyl-5-methyl-6-oxo-5,6,7,8-tetrahydro-pteridin-2-ylamino)-N-[4-(4-ethyl-piperazin-1-yl)-phenyl]-3-methoxy-benzamide |
| 10 | 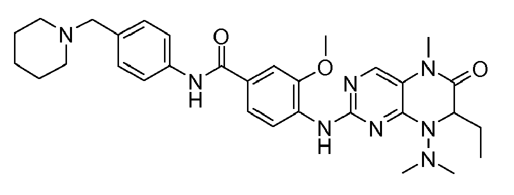 | 4-(8-Dimethylamino-7-ethyl-5-methyl-6-oxo-5,6,7,8-tetrahydro-pteridin-2-ylamino)-3-methoxy-N-(4-piperidin-1-ylmethyl-phenyl)-benzamide |
| 11 | 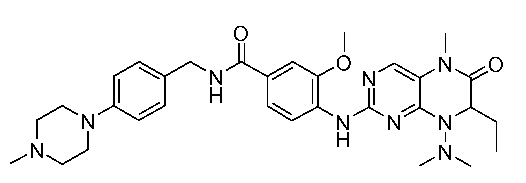 | 4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-benzyl]-benzamide |
| 12 | 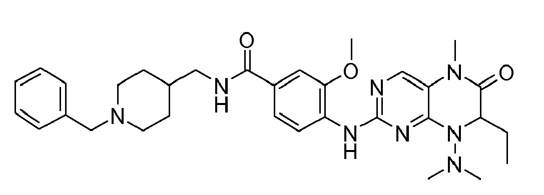 | N-(1-Benzyl-piperidin-4-ylmethyl)-4-(8-dimethylamino-7-ethyl-5-methyl-6-oxo-5,6,7,8-tetrahydro-pteridin-2-ylamino)-3-methoxy-benzamide |
| 13 | 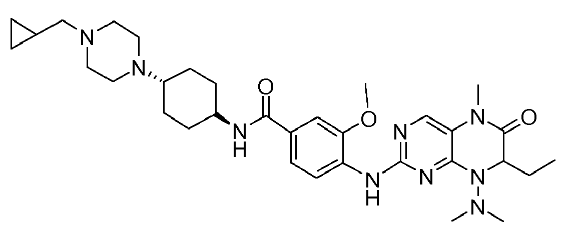 | N-[trans-4-(4-Cyclopropylmethyl-piperazin-1-yl)-cyclohexyl]-4-(8-dimethylamino-7-ethyl-5-methyl-6-oxo-5,6,7,8-tetrahydro-pteridin-2-ylamino)-3-methoxy-benzamide |
| 14 | 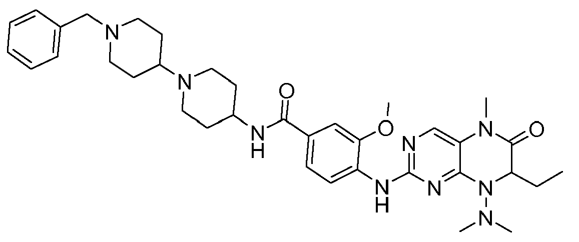 | N-(1'-Benzyl-[1,4']bipiperidinyl-4-yl)-4-(8-dimethylamino-7-ethyl-5-methyl-6-oxo-5,6,7,8-tetrahydro-pteridin-2-ylamino)-3-methoxy-benzamide |
| 15 | 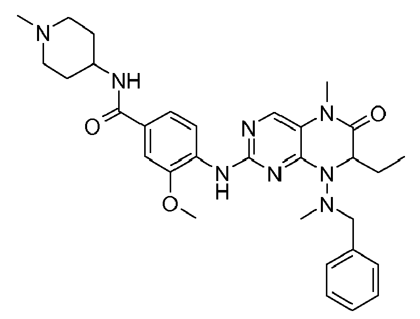 | 4-[8-(Benzyl-methyl-amino)-7-ethyl-5-methyl-6-oxo-5,6,7,8-tetrahydro-pteridin-2-ylamino]-3-methoxy-N-(1-methyl-piperidin-4-yl)-benzamide |
| 16 | 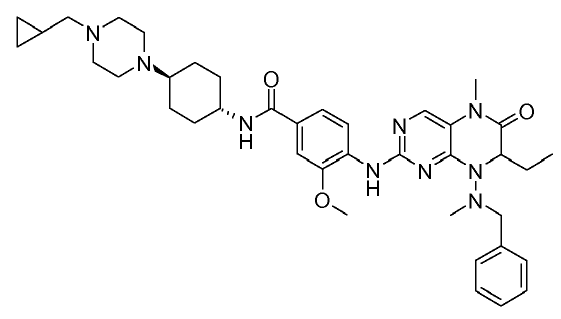 | 4-[8-(Benzyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)- cyclohexyl]-3-methoxy-benzamide |
| 17 | 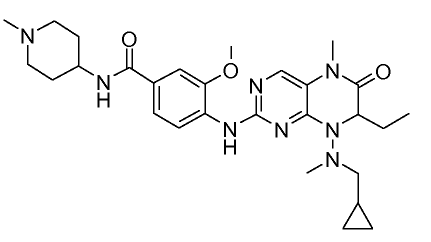 | 4-[8-(Cyclopropylmethyl-methyl-amino)-7-ethyl-5-methyl-6-oxo-5,6,7,8-tetrahydro-pteridin-2-ylamino]-3-methoxy-N-(1-methyl-piperidin-4-yl)-benzamide |
| 18 | 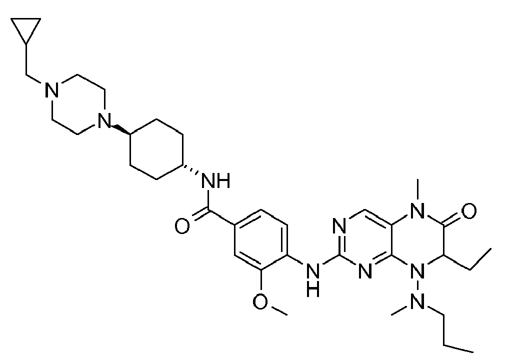 | 4-[8-(Cyclopropylmethyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)-cyclohexyl]-3-methoxy-benzamide |
| 19 | 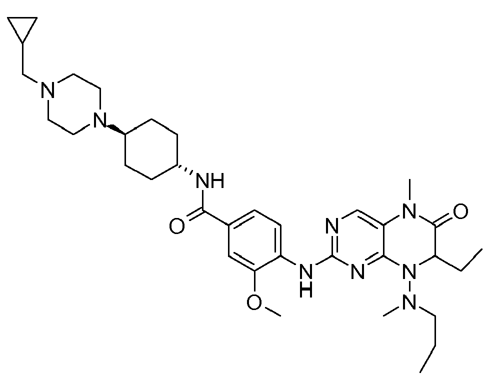 | N-[trans-4-(4-Cyclopropylmethyl-piperazin-1-yl)-cyclohexyl]-4-[7-ethyl-5-methyl-8-(methyl-propyl-amino)-6-oxo-5,6,7,8-tetrahydro-pteridin-2-ylamino]-3-methoxy-benzamide |
| 20 | 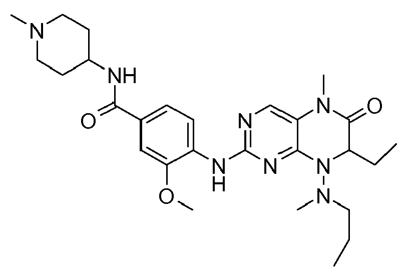 | 4-[7-Ethyl-5-methyl-8-(methyl-propyl-amino)-6-oxo-5,6,7,8-tetrahydro-pteridin-2-ylamino]-3-methoxy-N-(1-methyl-piperidin-4-yl)-benzamide |
| 21 | 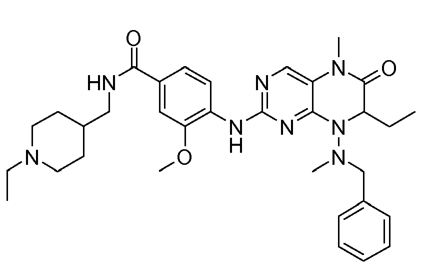 | 4-[8-(Benzyl-methyl-amino)-7-ethyl-5-methyl-6-oxo-5,6,7,8-tetrahydro-pteridin-2-ylamino]-N-(1-ethyl-piperidin-4-ylmethyl)-3-methoxy-benzamide |
| 22 | 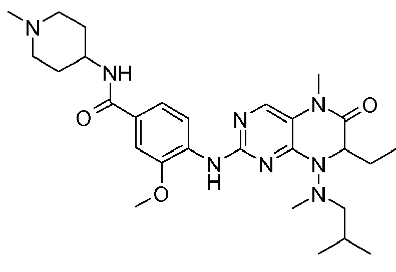 | 4-[7-Ethyl-8-(isobutyl-methyl-amino)-5-methyl-6-oxo-5,6,7,8-tetrahydro-pteridin-2-ylamino]-3-methoxy-N-(1-methyl-piperidin-4-yl)-benzamide |
| 23 | 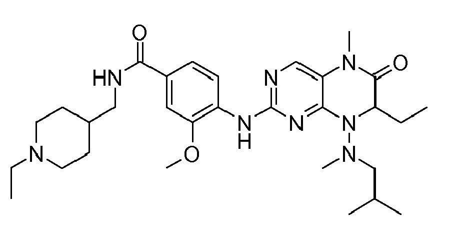 | 4-[7-Ethyl-8-(isobutyl-methyl-amino)-5-methyl-6-oxo-5,6,7,8-tetrahydro-pteridin-2-ylamino]-N-(1-ethyl-piperidin-4-ylmethyl)-3-methoxy-benzamide |
| 24 | 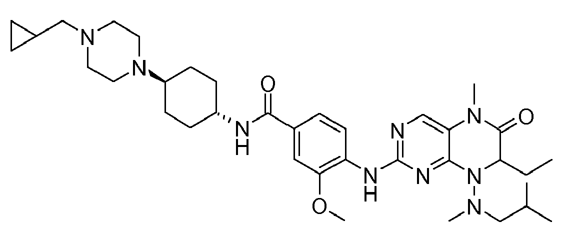 | N-[trans-4-(4-Cyclopropylmethyl-piperazin-1-yl)-cyclohexyl]-4-[7-ethyl-8-(isobutyl-methyl-amino)-5-methyl-6-oxo-5,6,7,8-tetrahydro-pteridin-2-ylamino]-3-methoxy-benzamide |
| 25 | 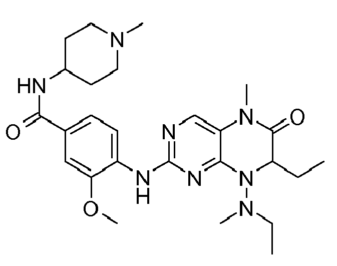 | 4-[7-Ethyl-8-(ethyl-methyl-amino)-5-methyl-6-oxo-5,6,7,8-tetrahydro-pteridin-2-ylamino]-3-methoxy-N-(1-methyl-piperidin-4-yl)-benzamide |
| 26 | 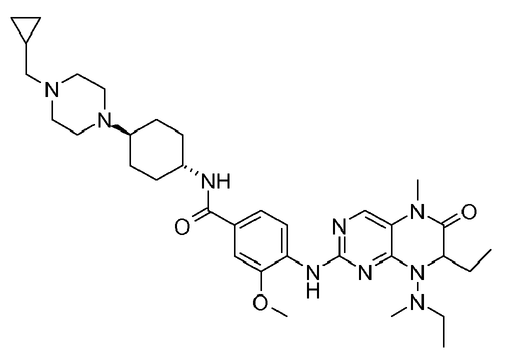 | N-[trans-4-(4-Cyclopropylmethyl-piperazin-1-yl)-cyclohexyl]-4-[7-ethyl-8-(ethyl-methyl-amino)-5-methyl-6-oxo-5,6,7,8-tetrahydro-pteridin-2-ylamino]-3-methoxy-benzamide |
| 27 | 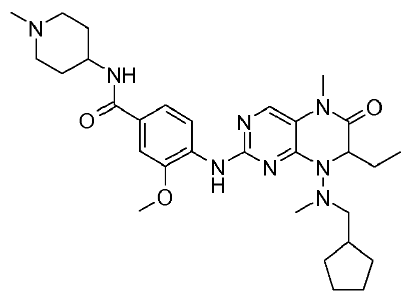 | 4-[8-(Cyclopentylmethyl-methyl-amino)-7-ethyl-5-methyl-6-oxo-5,6,7,8-tetrahydro-pteridin-2-ylamino]-3-methoxy-N-(1-methyl-piperidin-4-yl)-benzamide |
| 28 | 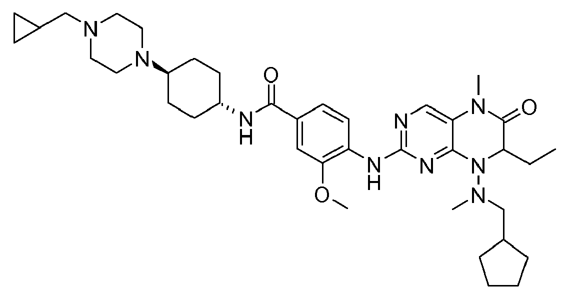 | 4-[8-(Cyclopentylmethyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)-cyclohexyl]-3-methoxy-benzamide |
| 29 | 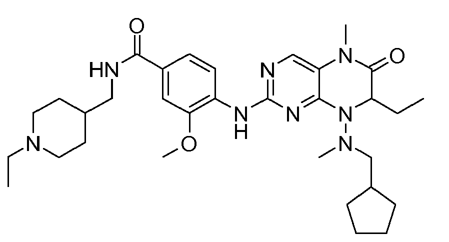 | 4-[8-(Cyclopentylmethyl-methyl-amino)-7-ethyl-5-methyl-6-oxo-5,6,7,8-tetrahydro-pteridin-2-ylamino]-N-(1-ethylpiperidin-4-ylmethyl)-3-methoxy-benzamide |
| 30 | 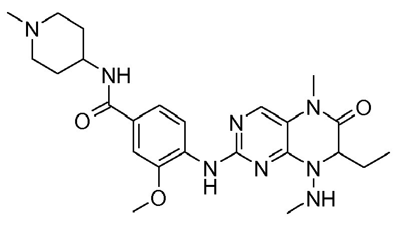 | 4-(7-Ethyl-5-methyl-8-methylamino-6-oxo-5,6,7,8-tetrahydro-pteridin-2-ylamino)-3-methoxy-N-(1-methyl-piperidin-4-yl)-benzamide |
| 31 | 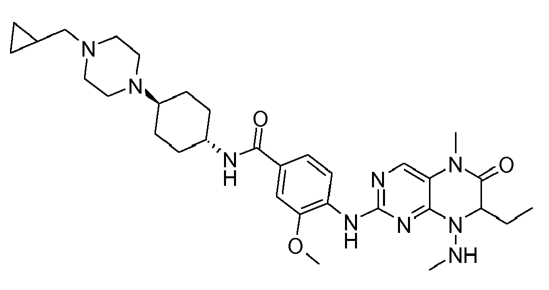 | N-[trans-4-(4-Cyclopropylmethyl-piperazin-1-yl)-cyclohexyl]-4-(7-ethyl-5-methyl-8-methylamino-6-oxo-5,6,7,8-tetrahydro-pteridin-2-ylamino)-3-methoxy-benzamide |
| 32 | 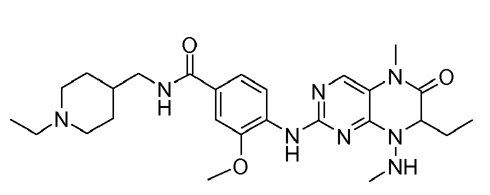 | 4-(7-Ethyl-5-methyl-8-methylamino-6-oxo-5,6,7,8-tetrahydro-pteridin-2-ylamino)-N-(1-ethyl-piperidin-4-ylmethyl)-3-methoxy-benzamide |
| 33 | 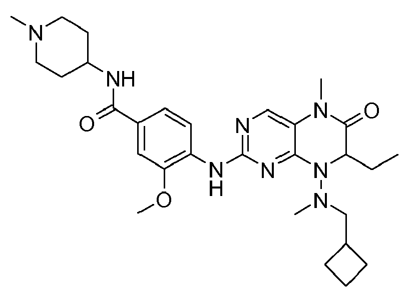 | 4-[8-(Cyclobutylmethyl-methyl-amino)-7-ethyl-5-methyl-6-oxo-5,6,7,8-tetrahydro-pteridin-2-ylamino]-3-methoxy-N-(1-methyl-piperidin-4-yl)-benzamide |
| 34 | 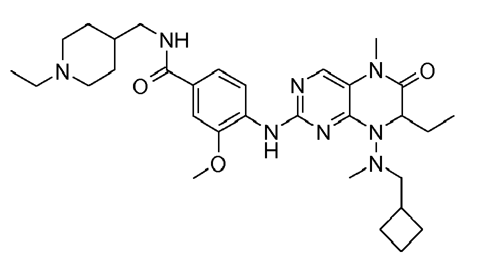 | 4-[8-(Cyclobutylmethyl-methyl-amino)-7-ethyl-5-methyl-6-oxo-5,6,7,8-tetrahydro-pteridin-2-ylamino]-N-(1-ethyl-piperidin-4-ylmethyl)-3-methoxy-benzamide |
| 35 | 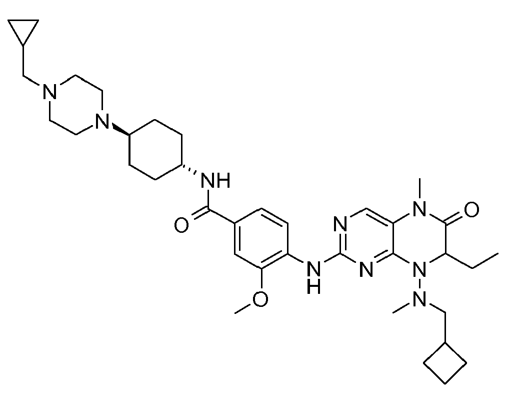 | 4-[8-(Cyclobutylmethyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)-cyclohexyl]-3-methoxy-benzamide |
| 36 | 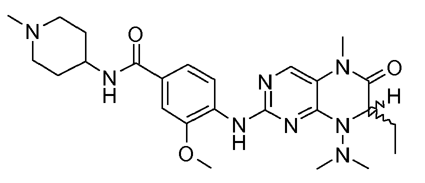 | Enantiomer 1 of 4-(8-dimethylamino-7-ethyl-5-methyl-6-oxo-5,6,7,8-tetrahydro-pteridin-2-ylamino)-3-methoxy-N-(1-methyl-piperidin-4-yl)-benzamide |
| 37 | 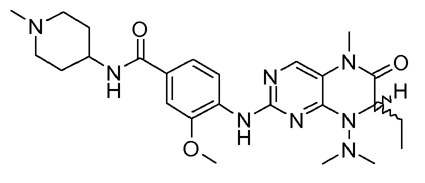 | Enantiomer 2 of 4-(8-dimethylamino-7-ethyl-5-methyl-6-oxo-5,6,7,8-tetrahydro-pteridin-2-ylamino)-3-methoxy-N-(1-methyl-piperidin-4-yl)-benzamide |

**Table 2: Characterization of exemplary compounds**

| Compound number | MH+ | Retention time. | Method |
|---|---|---|---|
| 1 | 497 | 3.98 | A |
| 2 | 497 | 5.52 | B |
| 3 | 525 | 5.56 | B |
| 4 | 525 | 5.60 | B |
| 5 | 511 | 5.65 | B |
| 6 | 527 | 5.82 | B |
| 7 | 574 | 5.88 | B |
| 8 | 588 | 5.94 | B |
| 9 | 573 | 6.05 | B |
| 10 | 588 | 5.87 | B |
| 11 | 587 | 5.96 | B |
| 12 | 587 | 5.96 | B |
| 13 | 620 | 3.96 | A |
| 14 | 656 | 3.90 | A |
| 15 | 573 | 4.78 | A |
| 16 | 696 | 4.61 | A |
| 17 | 537 | 4.32 | A |
| 18 | 660 | 4.36 | A |
| 19 | 648 | 4.41 | A |
| 20 | 525 | 4.46 | A |
| 21 | 601 | 4.88 | A |
| 22 | 539 | 4.67 | A |
| 23 | 567 | 4.80 | A |
| 24 | 662 | 4.59 | A |
| 25 | 511 | 4.24 | A |
| 26 | 634 | 4.16 | A |
| 27 | 565k | 5.06 | A |
| 28 | 688 | 4.89 | A |
| 29 | 693 | 5.09 | A |
| 30 | 483 | 3.73 | A |
| 31 | 606 | 3.76 | A |
| 32 | 511 | 3.87 | A |
| 33 | 551 | 4.69 | A |
| 34 | 579 | 4.74 | A |
| 35 | 664 | 4.59 | A |
| 36 | | See Example 4 | |
| 37 | | See Example 4 | |

**Table 3: IC₅₀ values of exemplary compounds in nucleophosmin assay (column 2); and inhibition of tumor cell growth (columns 3/5)**

| Compound number | Biochemical IC₅₀ [µM] Nucleophosmin assay | CELLULAR IC₅₀ [ M] | | |
|---|---|---|---|---|
| | | HCT 116 | HT-29 | IMR-90 |
| 1 | < 0.1 | < 1 | < 1 | > 1 |
| 2 | < 0.1 | < 1 | < 1 | > 1 |
| 3 | < 0.1 | < 1 | < 1 | > 1 |
| 4 | < 0.1 | < 1 | < 1 | > 1 |
| 5 | < 0.1 | < 1 | < 1 | > 1 |
| 6 | < 0.1 | < 1 | < 1 | > 1 |
| 7 | < 0.1 | < 1 | < 1 | > 1 |
| 8 | < 0.1 | < 1 | < 1 | > 1 |
| 9 | < 0.1 | < 1 | < 1 | > 1 |
| 10 | < 0.1 | < 1 | < 1 | > 1 |
| 11 | < 0.1 | < 1 | < 1 | > 1 |
| 12 | < 0.1 | < 1 | < 1 | > 1 |
| 13 | < 0.1 | < 1 | < 1 | > 1 |
| 14 | < 0.1 | < 1 | < 1 | > 1 |
| 15 | < 0.1 | < 1 | < 1 | > 1 |
| 16 | < 0.1 | < 1 | < 1 | > 1 |
| 17 | < 0.5 | < 1 | < 1 | > 1 |
| 18 | < 0.5 | < 1 | < 1 | > 1 |
| 19 | < 0.1 | < 1 | < 1 | > 1 |
| 20 | < 0.1 | < 1 | < 1 | > 1 |
| 21 | < 0.5 | < 1 | < 1 | > 1 |
| 22 | < 0.1 | < 1 | < 1 | > 1 |
| 23 | < 0.1 | < 1 | < 1 | > 1 |
| 24 | < 0.1 | < 1 | < 1 | > 1 |
| 25 | < 0.1 | < 1 | < 1 | > 1 |
| 26 | < 0.1 | < 1 | < 1 | > 1 |
| 27 | < 0.1 | < 1 | < 1 | > 1 |
| 28 | < 0.1 | < 1 | < 1 | > 1 |
| 29 | < 0.1 | < 1 | < 1 | > 1 |
| 30 | < 0.1 | < 1 | < 1 | > 1 |
| 31 | < 0.1 | < 1 | < 1 | > 1 |
| 32 | < 0.5 | < 1 | < 1 | > 1 |
| 33 | < 0.1 | < 1 | < 1 | > 1 |
| 34 | < 0.5 | < 1 | < 1 | > 1 |
| 35 | < 0.5 | < 1 | < 1 | > 1 |
| 36 | > 1 | > 1 | > 1 | > 1 |
| 37 | < 0.1 | < 1 | < 1 | > 1 |

### Table 4: Substitution pattern at R¹ for exemplary compounds:

### Compound 7:

Substituent R¹: substituted aryl 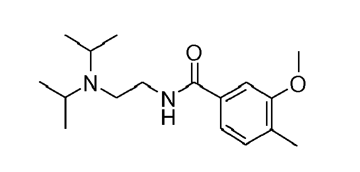
Aryl =phenyl
Substituent A (o-OMe) = -[(R^{Sm})ₙ] with n = 1: R^{S1} = -O-alkyl
Substituent B (p-carboxamido) = -[(R^{Sm})ₙ] with n = 3: R^{S1} = -C(=O)N(R^{a})₂; R^{S2} = -alkyl; R^{S3} = - N(R^{a})₂

### Compound 8:

Substituent R¹: substituted aryl 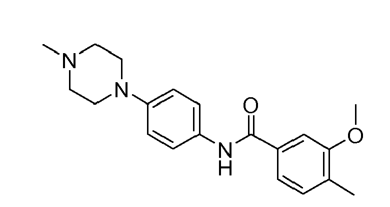
Aryl =phenyl
Substituent A (o-OMe) = -[(R^{Sm})ₙ] with n = 1: R^{S1} = -O-alkyl
Substituent B (p-carboxamido) = -[(R^{Sm})ₙ] with n = 4: R^{S1} = -C(=O)N(R^{a})₂; R^{S2} = -aryl; R^{S3} = - heterocycloalkyl; R^{S4} = -alkyl

### Compound 13:

Substituent R¹: substituted aryl 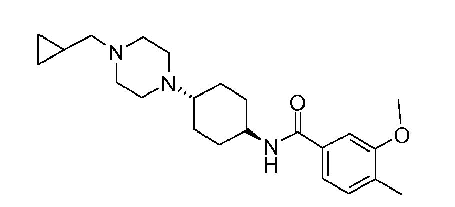
Aryl =phenyl
Substituent A (o-OMe) = -[(R^{Sm})ₙ] with n = 1: R^{S1} = -O-alkyl
Substituent B (p-carboxamido) = -[(R^{Sm})ₙ] with n = 5: R^{S1} = -C(=O)N(R^{a})₂; R^{S2} = -cycloalkyl; R^{S3} = - heterocycloalkyl; R^{S4} = -alkyl; R^{S5} = -cycloalkyl;

### Equivalents

Those skilled in the art will recognize, or be able to ascertain using no more than routine experimentation, many equivalents to the specific embodiments of the invention described herein. Such equivalents are intended to be encompassed by the following claims.

All of the above-cited references and publications are hereby incorporated by reference.

## Claims

1. A compound having a structure represented by formula (I) 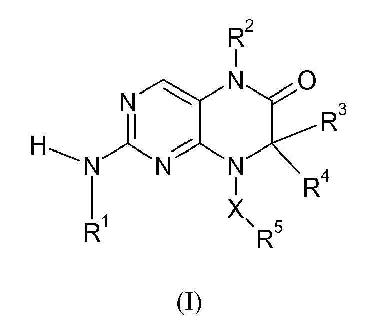 or any tautomeric or stereoisomeric form thereof, wherein
R¹ is selected from substituted or unsubstituted: -aryl and -heteroaryl;
R² is selected from hydrogen, -CH(=O), -OH, substituted or unsubstituted -O-C₁₋₂-alkyl and substituted or unsubstituted -C₁₋₆-alkyl;
R³ and R⁴ are independently selected from hydrogen, and substituted or unsubstituted: -C₁₋₁₀-alkyl, -C₂₋₁₀-alkenyl, -C₂₋₁₀-alkynyl, -C₃₋₁₀-cycloalkyl, -C₄₋₁₀-cycloalkenyl, heterocycloalkyl, heterocycloalkenyl, aryl, and heteroaryl;
alternatively, R³ and R⁴, together with the carbon atom to which they are attached, form a substituted or unsubstituted: C₃₋₈-cycloalkyl, C₄₋₈-cycloalkenyl or a substituted or unsubstituted: 4- to 8-membered heterocycloalkyl or heterocycloalkenyl comprising 1, 2, or 3 heteroatoms selected from N and O;
X is selected from -O- and -NR⁶;
R⁵ and R⁶ are independently selected from hydrogen, and substituted or unsubstituted: -C₁₋₆-alkyl, -C₂₋₆-alkenyl, -C₂₋₆-alkynyl, -C₃₋₆-cycloalkyl and -C₄₋₆-cycloalkenyl;
alternatively, for X = NR⁶, R⁵ and R⁶, together with the nitrogen atom they are attached to, form a substituted or unsubstituted: 4- to 8-membered heterocycloalkyl or heterocycloalkenyl comprising 0, 1, or 2 additional heteroatoms selected from N and O;
alternatively, one of R³ and R⁴, and R⁵, together with the group -O-N-C-, or -N-N-C, respectively, to which they are attached, form a substituted or unsubstituted: 4- to 8-membered heterocycloalkyl or heterocycloalkenyl comprising 0 or 1 additional heteroatom selected from N and O;
or any pharmaceutically acceptable salt or N-oxide thereof.

2. The compound of claim 1, wherein R¹ is substituted or unsubstituted phenyl.

3. The compound of claim 2, wherein R¹ is phenyl substituted with 1, 2, or 3 substituents R⁷, wherein each R⁷ is independently selected from the list of: -CN, -NO₂, -N₃, -F, -Cl, -Br, -I, and substituted or unsubstituted: alkyl, alkenyl, alkynyl, cycloalkyl, cycloalkenyl, C-linked heterocycloalkyl, C-linked hetero-cycloalkenyl, aryl, C-linked heteroaryl, -C(=O)-R^{a}, -C(=O)-OR^{a}, -C(=O)-N(R^{a})₂, -O-R^{a}, -S-R^{a}, -SO-R^{a}-SO₂-R^{a}, -SO₂-N(R^{a})₂, N-linked heterocycloalkyl, N-linked heteroaryl, -N(R^{a})-R^{a}, -N(R^{a})-OR^{a}, -N(R^{a})-N(R^{a})₂, -N(R^{a})-C(=O)-R^{a}, -N(R^{a})-C(=O)-OR^{a}, -N(R^{a})-C(=O)-N(R^{a})₂, and -N(R^{a})-SO₂-R^{a}, wherein each R^{a} is independently selected from -H and R^{b}, with R^{b} being independently selected from alkyl, alkenyl, alkynyl, cycloalkyl, cycloalkenyl, C-linked heterocycloalkyl, C-linked heterocycloalkenyl, aryl, and C-linked heteroaryl; alternatively, two substituents R^{b}, when attached to the same nitrogen atom, together with the nitrogen atom to which they are attached, form a heterocycloalkyl, heterocycloalkenyl, or heteroaryl.

4. The compound of claim 3, wherein R¹ is phenyl substituted with at least one substituent R⁷ in position 4.

5. The compound of claim 3 or 4, wherein R⁷ is -Y-R⁸, wherein Y is selected from the list of -C(=O)NH-, -NH-C(=O)- , -SO₂-NH-, and -NH-SO₂-, and wherein R⁸ is selected from the list of substituted or unsubstituted: alkyl, alkenyl, alkynyl, cycloalkyl, cycloalkenyl, C-linked heterocycloalkyl, C-linked heterocycloalkenyl, aryl, and C-linked heteroaryl.

6. The compound of any one of claims 1 to 5, wherein R² is substituted or unsubstituted -C₁₋₆-alkyl.

7. The compound of claim 6, wherein R² is methyl.

8. The compound of any one of claims 1 to 7, wherein R³ and R⁴ are independently selected from hydrogen, and substituted or unsubstituted -C₁₋₁₀-alkyl.

9. The compound of claim 8, wherein one of R³ and R⁴ is hydrogen, and the other is ethyl.

10. The compound of any one of claims 1 to 9 wherein X is -NR⁶.

11. The compound of claim 10, wherein R⁵ and R⁶ are independently selected from hydrogen, and substituted or unsubstituted -C₁₋₆-alkyl.

12. The compound of claim 11, wherein R⁵ is methyl, and R⁶ is selected from substituted or unsubstituted -C₁₋₆-alkyl.

13. The compound of claim 5 having a structure represented by formula (Ib) 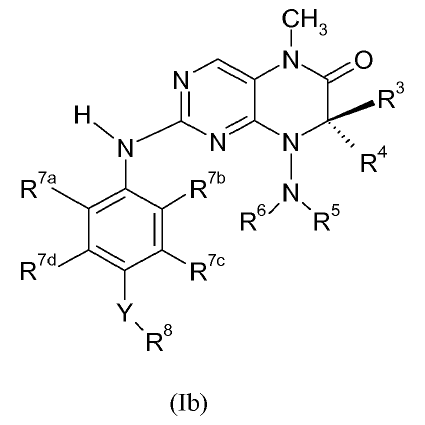 or any tautomeric or stereoisomeric form thereof, wherein
R^{7a} is selected from the list of: -hydrogen, -CN, -F, -Cl, -Br, -I, and substituted or unsubstituted: -C₁₋₆-alkyl, -C₁₋₆-alkenyl, -C₁₋₆-alkynyl, -O-R^{c}, -S-R^{d}, -SO-R^{d}, -SO₂-R^{d}, and -N(R^{c})-R^{c}, wherein R^{c} is selected from the list of: hydrogen and R^{d}, and wherein R^{d} is selected from C₁₋₆-alkyl, C₁₋₆-alkenyl, and C₁₋₆-alkynyl; and
R^{7b} , R^{7c} and R^{7d} are each independently selected from hydrogen, -F, -Cl, -Br, -I, -methyl, -CH₂F, -CHF₂, -CF₃, -O-methyl, -O-CH₂F, -O-CHF₂, and -O-CF₃.

14. The compound of claim 13, wherein Y is -C(=O)-NH-.

15. The compound of claim 14, wherein R⁸ is N-methylpiperidin-4-yl.

16. The compound of any one of claims 13 to 15, wherein R^{7a} is substituted or unsubstituted -O-C₁₋₃-alkyl.

17. The compound of any one of claims 13 to 16, wherein R³ and R⁴ are independently selected from hydrogen, and substituted or unsubstituted -C₁₋₁₀-alkyl.

18. The compound of claim 17, wherein one of R³ and R⁴ is hydrogen, and the other is ethyl.

19. The compound of any one of claims 13 to 18, wherein R⁵ and R⁶ are independently selected from hydrogen, and substituted or unsubstituted -C₁₋₆-alkyl.

20. The compound of claim 19, wherein R⁵ is methyl, and R⁶ is selected from substituted or unsubstituted -C₁₋₆-alkyl.

21. The compound of claim 1, wherein the compound is selected from:
4-(8-Dimethylamino-7-ethyl-5-methyl-6-oxo-5,6,7,8-tetrahydro-pteridin-2-ylamino)-3-methoxy-N-(1-methyl-piperidin-4-yl)-benzamide;
4-(8-Dimethylamino-7-ethyl-5-methyl-6-oxo-5,6,7,8-tetrahydro-pteridin-2-ylamino)-3-methoxy-N-(2-pyrrolidin-1-yl-ethyl)-benzamide;
4-(8-Dimethylamino-7-ethyl-5-methyl-6-oxo-5,6,7,8-tetrahydro-pteridin-2-ylamino)-N-(1-ethyl-piperidin-4-ylmethyl)-3-methoxy-benzamide;
4-(8-Dimethylamino-7-ethyl-5-methyl-6-oxo-5,6,7,8-tetrahydro-pteridin-2-ylamino)-N-(1-isopropyl-piperidin-4-yl)-3-methoxy-benzamide;
N-(2-Diethylamino-ethyl)-4-(8-dimethylamino-7-ethyl-5-methyl-6-oxo-5,6,7,8-tetrahydro-pteridin-2-ylamino)-3-methoxy-benzamide;
4-(8-Dimethylamino-7-ethyl-5-methyl-6-oxo-5,6,7,8-tetrahydro-pteridin-2-ylamino)-3-methoxy-N-(2-piperidin-1-yl-ethyl)-benzamide;
N-(2-Diisopropylamino-ethyl)-4-(8-dimethylamino-7-ethyl-5-methyl-6-oxo-5,6,7,8-tetrahydro-pteridin-2-ylamino)-3-methoxy-benzamide;
4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-phenyl]-benzamide;
4-(8-Dimethylamino-7-ethyl-5-methyl-6-oxo-5,6,7,8-tetrahydro-pteridin-2-ylamino)-N-[4-(4-ethyl-piperazin-1-yl)-phenyl]-3-methoxy-benzamide;
4-(8-Dimethylamino-7-ethyl-5-methyl-6-oxo-5,6,7,8-tetrahydro-pteridin-2-ylamino)-3-methoxy-N-(4-piperidin-1-ylmethyl-phenyl)-benzamide;
4-(8-Dimethylamino-7-ethyl-5-methyl-6-oxo-5,6,7,8-tetrahydro-pteridin-2-ylamino)-3-methoxy-N-[4-(4-methyl-piperazin-1-yl)-benzyl]-benzamide;
N-(1-Benzyl-piperidin-4-ylmethyl)-4-(8-dimethylamino-7-ethyl-5-methyl-6-oxo-5,6,7,8-tetrahydro-pteridin-2-ylamino)-3-methoxy-benzamide;
N-[trans-4-(4-Cyclopropylmethyl-piperazin-1-yl)-cyclohexyl]-4-(8-dimethylamino-7-ethyl-5-methyl-6-oxo-5,6,7,8-tetrahydro-pteridin-2-ylamino)-3-methoxy-benzamide;
N-(1'-Benzyl-[1,4']bipiperidinyl-4-yl)-4-(8-dimethylamino-7-ethyl-5-methyl-6-oxo-5,6,7,8-tetrahydro-pteridin-2-ylamino)-3-methoxy-benzamide;
4-[8-(Benzyl-methyl-amino)-7-ethyl-5-methyl-6-oxo-5,6,7,8-tetrahydro-pteridin-2-ylamino]-3-methoxy-N-(1-methyl-piperidin-4-yl)-benzamide;
4-[8-(Benzyl-methyl-amino)-7-ethyl-5-methyl-6-oxo-5,6,7,8-tetrahydro-pteridin-2-ylamino]-N-[trans-4-(4-cyclopropylmethyl-piperazin-1-yl)-cyclohexyl]-3-methoxy-benzamide;
4-[8-(Cyclopropylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide;
4-[8-(Cyclopropylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide;
N-[trans-4-(4-Cyclopropylmethyl-piperazin-1-yl)-cyclohexyl]-4-[7-ethyl-5-methyl-8-(methyl-propyl-amino)-6-oxo-5,6,7,8-tetrahydro-pteridin-2-ylamino]-3-methoxy-benzamide;
4-[7-Ethyl-5-methyl-8-(methyl-propyl-amino)-6-oxo-5,6,7,8-tetrahydro-pteridin-2-ylamino]-3-methoxy-N-(1-methyl-piperidin-4-yl)-benzamide;
4-[8-(Benzyl-methyl-amino)-7-ethyl-5-methyl-6-oxo-5,6,7,8-tetrahydro-pteridin-2-ylamino]-N-(1-ethyl-piperidin-4-ylmethyl)-3-methoxy-benzamide;
4-[7-Ethyl-8-(isobutyl-methyl-amino)-5-methyl-6-oxo-5,6,7,8-tetrahydro-pteridin-2-ylamino]-3-methoxy-N-(1-methyl-piperidin-4-yl)-benzamide;
4-[7-Ethyl-8-(isobutyl-methyl-amino)-5-methyl-6-oxo-5,6,7,8-tetrahydro-pteridin-2-ylamino]-N-(1-ethyl-piperidin-4-ylmethyl)-3-methoxy-benzamide;
N-[trans-4-(4-Cyclopropylmethyl-piperazin-1-yl)-cyclohexyl]-4-[7-ethyl-8-(isobutylmethyl-amino)-5-methyl-6-oxo-5,6,7,8-tetrahydro-pteridin-2-ylamino]-3-methoxy-benzamide;
4-[7-Ethyl-8-(ethyl-methyl-amino)-5-methyl-6-oxo-5,6,7,8-tetrahydro-pteridin-2-ylamino]-3-methoxy-N-(1-methyl-piperidin-4-yl)-benzamide;
N-[trans-4-(4-Cyclopropylmethyl-piperazin-1-yl)-cyclohexyl]-4-[7-ethyl-8-(ethylmethyl-amino)-5-methyl-6-oxo-5,6,7,8-tetrahydro-pteridin-2-ylamino]-3-methoxy-benzamide;
4-[8-(Cyclopentylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide;
4-[8-(Cyclopentylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide;
4-[8-(Cyclopentylmethyl-methyl-amino)-7-ethyl-5-methyl-6-oxo-5,6,7,8-tetrahydropteridin-2-ylamino]-N-(1-ethyl-piperidin-4-ylmethyl)-3-methoxy-benzamide;
4-(7-Ethyl-5-methyl-8-methylamino-6-oxo-5,6,7,8-tetrahydro-pteridin-2-ylamino)-3-methoxy-N-(1-methyl-piperidin-4-yl)-benzamide;
N-[trans-4-(4-Cyclopropylmethyl-piperazin-1-yl)-cyclohexyl]-4-(7-ethyl-5-methyl-8-methylamino-6-oxo-5,6,7,8-tetrahydro-pteridin-2-ylamino)-3-methoxy-benzamide;
4-(7-Ethyl-5-methyl-8-methylamino-6-oxo-5,6,7,8-tetrahydro-pteridin-2-ylamino)-N-(1-ethyl-piperidin-4-ylmethyl)-3-methoxy-benzamide;
4-[8-(Cyclobutylmethyl-methyl-amino)-7-ethyl-5-methyl-6-oxo-5,6,7,8-tetrahydropteridin-2-ylamino]-3-methoxy-N-(1-methyl-piperidin-4-yl)-benzamide
4-[8-(Cyclobutylmethyl-methyl-amino)-7-ethyl-5-methyl-6-oxo-5,6,7,8-tetrahydropteridin-2-ylamino]-N-(1-ethyl-piperidin-4-ylmethyl)-3-methoxy-benzamide; and
4-[8-(Cyclobutylmethyl-methyl-amino)-7-ethyl-5-methyl-6-oxo-5,6,7,8-tetrahydropteridin-2-ylamino]-N-[trans-4-(4-cyclopropylmethyl-piperazin-1-yl)-cyclohexyl]-3-methoxy-benzamide.

22. A pharmaceutical composition, including a compound of any one of claims 1 to 21, and a pharmaceutically acceptable diluent, excipient or carrier.
